# EUROPEAN PATENT SPECIFICATION

(11) **EP 1 599 573 B1**
(45) Date of publication and mention of the grant of the patent: **19.06.2013**
(21) Application number: 04711868.2
(22) Date of filing: 17.02.2004
(51) Int. Cl.: C12N 9/12, C12N 15/00, C12N 15/63, A01N 43/04, A01N 63/00

(54) **MODEL FOR STUDYING THE ROLE OF GENES IN TUMOR RESISTANCE TO CHEMOTHERAPY**
MODELL ZUR UNTERSUCHUNG DER ROLLE VON GENEN BEI TUMORRESISTENZ IN DER CHEMOTHERAPIE
MODELE D'ETUDE DU ROLE DE GENES DANS LA RESISTANCE DE TUMEUR A LA CHIMIOTHERAPIE

(30) Priority: 17.02.2003 US 448198 P; 30.05.2003 US 474742 P; 04.02.2004 US 542010 P
(43) Date of publication of application: 30.11.2005
(73) Proprietor: COLD SPRING HARBOR LABORATORY, Cold Spring Harbor, NY 11724 (US); McGill University, Montreal, QC H3A 2T5 (CA)
(72) Inventor: FRIDMAN, Jordan S., Bear, DE 19701 (US); LOWE, Scott, Cold Spring Harbor, NY 11724 (US); DE STANCHINA, Elisa, Huntington Station, NY 11746 (US); WENDEL, Hans G., Huntington, NY 11743 (US); PELLETIER, Jerry, Baie d'Urfe, Québec H9X 3B1 (CA)
(74) Representative: Evenson, Jane Harriet
(86) International application number: PCT/US2004/004686
(87) International publication number: WO 2004/074445

(56) References cited:
- WO-A2-00/20025
- SCHMITT CLEMENS A ET AL: "Dissecting p53 tumor suppressor functions in vivo" CANCER CELL, vol. 1, no. 3, April 2002 (2002-04), pages 289-298, XP002438822 ISSN: 1535-6108
- SCHMITT CLEMENS A ET AL: "A senescence program controlled by p53 and p16INK4a contributes to the outcome of cancer therapy" CELL, vol. 109, no. 3, 3 May 2002 (2002-05-03), pages 335-346, XP002438823 ISSN: 0092-8674
- DE MIGUEL MARIA P ET AL: "Dissection of the c-Kit signaling pathway in mouse primordial germ cells by retroviral-mediated gene transfer" PROCEEDINGS OF THE NATIONAL ACADEMY OF SCIENCES OF THE UNITED STATES OF AMERICA, vol. 99, no. 16, 6 August 2002 (2002-08-06), pages 10458-10463, XP002438824 ISSN: 0027-8424
- MANGI ABEEL A ET AL: "GENETICALLY MODIFIED MESENCHYMAL STEM CELLS PERFORM IN VIVO REPAIR OF DAMAGED MYOCARDIUM" CIRCULATION, AMERICAN HEART ASSOCIATION, DALLAS, TX, US, vol. 106, no. 19 SUPPL, 5 November 2002 (2002-11-05), pages II-131, XP008072745 ISSN: 0009-7322
- WEST K A ET AL: "Activation of the PI3K/Akt pathway and chemotherapeutic resistance" DRUG RESISTANCE UPDATES, CHURCHILL LIVINGSTONE, EDINBURGH, GB, vol. 5, no. 6, December 2002 (2002-12), pages 234-248, XP002964818 ISSN: 1368-7646
- NERI LUCA M ET AL: "The phosphoinositide 3-kinase/AKT1 pathway involvement in drug and all-trans-retinoic acid resistance of leukemia cells." MOLECULAR CANCER RESEARCH, vol. 1, no. 3, January 2003 (2003-01), pages 234-246, XP002438825 ISSN: 1541-7786
- BORTUL R ET AL: "Constitutively active Akt1 protects HL60 leukemia cells from TRAIL-induced apoptosis through a mechanism involving NF-kappaB activation and cFLIP(L) up-regulation." LEUKEMIA : OFFICIAL JOURNAL OF THE LEUKEMIA SOCIETY OF AMERICA, LEUKEMIA RESEARCH FUND, U.K FEB 2003, vol. 17, no. 2, February 2003 (2003-02), pages 379-389, XP002438826 ISSN: 0887-6924
- MANGI ABEEL A ET AL: "Mesenchymal stem cells modified with Akt prevent remodeling and restore performance of infarcted hearts" NATURE MEDICINE, NATURE PUBLISHING GROUP, NEW YORK, NY, US, vol. 9, no. 9, September 2003 (2003-09), pages 1195-1201, XP002358429 ISSN: 1078-8956
- AOKI M. ET AL: 'The akt kinase: molecular determinants of oncogenicity' PROC. NATL. ACAD. SCI. USA vol. 95, December 1998, pages 14950 - 14955, XP002996032
- DUPRAZ P. ET AL: 'Lentivirus-mediated Bcl-2 expression in betaTC-tet cells improves resistance to hypoxia and cytokine-induced apoptosis while preserving in vitro and in vivo control of insulin secretion' GENE THER. vol. 6, June 1999, pages 1160 - 1169, XP002996033
- NORRIS P.S. ET AL: 'High-titer MSCV-based retrovirus generated in the pCL acute virus packaging system confers sustained gene expression in vivo' J. VIROL METHODS vol. 75, November 1998, pages 161 - 167, XP002996034
- WENDEL H.G. ET AL: 'Survival signalling by Akt and eIF4E in oncogenesis and cancer therapy' NATURE vol. 428, March 2004, pages 332 - 337, XP002996035
- SHI Y. ET AL: 'Enhanced sensitivity of multiple myeloma cells containing PTEN mutations to CCI-779' CANCER RES. vol. 62, September 2002, pages 5027 - 5034, XP002323911
- ASNAGHI L. ET AL: 'Bcl-2 phosphorylation and apoptosis activated by damaged microtubules require mTOR and are regulated by Akt' ONCOGENE vol. 23, July 2004, pages 5781 - 5791, XP002996036
- KUME A. ET AL: 'Green fluorescent protein as a selectable marker of retrovirally transduced hematopoietic progenitors' STEM CELLS vol. 17, 1999, pages 226 - 232, XP002996037
- DE BENEDETTI A ET AL: "eIF-4E expression and its role in malignancies and metastases", ONCOGENE, NATURE PUBLISHING GROUP, GB, vol. 23, no. 18, 1 April 2004 (2004-04-01) , pages 3189-3199, XP008105358, ISSN: 0950-9232, DOI: 10.1038/SJ.ONC.1207545
- LAZARIS-KARATZAS A ET AL: "MALIGNANT TRANSFORMATION BY A EUKARYOTIC INITIATION FACTOR SUBUNIT THAT BINDS TO MESSENGER RNA 5' CAP", NATURE (LONDON), vol. 345, no. 6275, 1990, pages 544-547, XP009163583, ISSN: 0028-0836
- TOPISIROVIC IVAN ET AL: "Aberrant eukaryotic translation initiation factor 4E-dependent mRNA transport impedes hematopoietic differentiation and contributes to leukemogenesis.", MOLECULAR AND CELLULAR BIOLOGY, vol. 23, no. 24, December 2003 (2003-12), pages 8992-9002, XP009163581, ISSN: 0270-7306
- MAYEUR G L ET AL: "Malignant transformation by the eukaryotic translation initiation factor 3 subunit p48 (eIF3e)", FEBS LETTERS, ELSEVIER, AMSTERDAM, NL, vol. 514, no. 1, 6 March 2002 (2002-03-06) , pages 49-54, XP004597948, ISSN: 0014-5793, DOI: 10.1016/S0014-5793(02)02307-4
- JEREMY R GRAFF ET AL: "Translational control and metastatic progression: Enhanced activity of the mRNA cap-binding protein eIF-4E selectively enhances translation of metastasis-related mRNAs", CLINICAL & EXPERIMENTAL METASTASIS ; OFFICIAL JOURNAL OF THEMETASTASIS RESEARCH SOCIETY, KLUWER ACADEMIC PUBLISHERS, DO, vol. 20, no. 3, 1 April 2003 (2003-04-01), pages 265-273, XP019235675, ISSN: 1573-7276, DOI: 10.1023/A:1022943419011
- SHE QING-BAI ET AL: "Resistance to gefitinib in PTEN-null HER-overexpressing tumor cells can be overcome through restoration of PTEN function or pharmacologic modulation of constitutive phosphatidylinositol 3'-kinase/Akt pathway signaling", CLINICAL CANCER RESEARCH, THE AMERICAN ASSOCIATION FOR CANCER RESEARCH, US, vol. 9, no. 12, 1 October 2003 (2003-10-01), pages 4340-4346, XP002597656, ISSN: 1078-0432
- WATKINS S J ET AL: "Translation initiation and its deregulation during tumorigenesis", BRITISH JOURNAL OF CANCER, vol. 86, no. 7, 8 April 2002 (2002-04-08), pages 1023-1027, XP009163579, ISSN: 0007-0920

### SEQUENCE LISTING

<110> COLD SPRING HARBOR LABORATORY McGILL UNIVERSITY
<120> MODEL FOR STUDYING THE ROLE OF GENES IN TUMOR RESISTANCE TO CHEMOTHERAPY
<130> P27841EP-PCT
<140> EP04711868.2 <141> 2004-02-17
<150> 60/448,198 <151> 2003-02-17
<150> 60/474,742 <151> 2003-05-30
<150> 60/542,010 <151> 2004-02-04
<160> 2
<170> PatentIn Ver. 3.2
<210> 1
   <211> 93
   <212> DNA
   <213> Mus musculus
<400> 1
   aaaaaagtca agtctaagcc gaatccaccc cctcgcaagc ttccaagagg atggattcga 60
   cttagacttg acggtgtttc gtcctttcca caa                              93
<210> 2
   <211> 70
   <212> DNA
   <213> Mus musculus
<400> 2
   aaaaaagggc cctgaattag aagaatacat cttcacaagc ttctgaagat atattcctcc 60
   aattcaggac                                                        70

## Description

### GOVERNMENT SUPPORT

The invention was supported, in whole or in part, by grants CA87497 and CA13106 from the National Cancer Institute. The Government has certain rights in the invention.

### CROSS-REFERENCE TO RELATED APPLICATIONS

This application claims priority to three U.S. Provisional Applications: Serial No. 60/448,198 filed February 17, 2003; Serial No. 60/474,742 filed May 30, 2003; and Serial No. 60/xxx,xxx filed February 4, 2004 (entitled "Model for Studying the Role of Genes in Tumor Resistance to Chemotherapy").

### FIELD OF THE INVENTION

The present disclosure is directed to vectors, cells and mammals, and methods which in part depend on such products, useful for understanding tumorigenesis and its treatments, and in particular, for identifying and studying inhibitors and activators associated with tumor cell growth and growth inhibition, cell death through apoptotic pathways, and changes in apoptotic pathway components that affect drug sensitivity and resistance in tumorigenic cells.

### BACKGROUND OF THE INVENTION

Resistance to cytotoxic agents used in cancer therapy remains a major obstacle in the treatment of human malignancies, including leukemia and lymphoma. Since most anticancer agents were discovered through empirical screens, efforts to overcome resistance are hindered by our limited understanding of why these agents are effective. The role of apoptosis in malignancy provides a new explanation for drug sensitivity and resistance. This view suggests that responsive tumors must readily undergo apoptosis in response to cytotoxic agents and that resistant tumors may have acquired mutations that suppress apoptosis. Also, the fact that apoptosis is controlled by genes raises the prospect that the problem of drug sensitivity and resistance will be amenable to molecular biology.

Apoptosis is controlled by a complex network of proliferation and survival genes that is frequently disrupted during tumor evolution. For example, the phosphatidylinositol 3-kinase (PI3K) pathway integrates receptor tyrosine kinase signaling with the apoptotic network (Datta, S. R., et al, "Cellular survival: a play in three Akts," Genes Dev. 13: 2905-2927 (1999) and Vivanco, I. and Sawyers, C. L., "The phosphatidylinositol 3-Kinase AKT pathway in human cancer," Nat. Rev. Cancer 2: 489-501 (2002)). One mediator of PI3K signaling is the Akt/PKB kinase, which phosphorylates multiple downstream effectors that ultimately produce global changes in cellular physiology. How Akt promotes survival is controversial, but may involve direct phosphorylation of apoptotic regulators, increased cell cycle progression, decreased transcription of pro-apoptotic genes via inhibition of forkhead transcription factors, altered metabolism, or changes in the translation of mRNAs that ultimately control cell death (Vivanco, I. and Sawyers, C. L., "The phosphatidylinositol 3-Kinase AKT pathway in human cancer," Nat. Rev. Cancer 2: 489-501 (2002)). Mutations that activate the PI3K/Akt pathway, including amplifications of PI3K pathway components and inactivation of the negative regulator PTEN are common in human malignancies (Steck, P. A., et al., "Identification of a candidate tumor suppressor gene, MMAC1, at chromosome 10q23.3 that is mutated in multiple advanced cancers," Nat. Genet. 15: 356-362 (1997); Sakai, A., et al., "PTEN gene alterations in lymphoid neoplasms," Blood 92: 3410-3415 (1998); and Min, Y. H., et al., "Constitutive phosphorylation of Akt/PKB protein in acute myeloid leukemia: its significance as a prognostic variable," Leukemia 17: 995-997 (2003)). Therefore, pharmacological agents that inhibit Akt or its crucial downstream effectors may be effective against many human cancers.

Evading apoptosis is considered a hallmark of cancer because mutations in apoptotic regulators invariably accompany tumorigenesis (Hanahan, D. and Weinberg, R. A., "The hallmarks of cancer," Cell 100: 57-70 (2000)). Many chemotherapeutic agents induce apoptosis, and so disruption of apoptosis during tumor evolution can promote drug resistance (Johnstone, R. W., et al., "Apoptosis: a link between cancer genetics and chemotherapy," Cell 108: 153-164 (2002)). Akt is an apoptotic regulator that is activated in many cancers and may promote drug resistance *in vitro* (Mayo, L. D., et al., "PTEN protects p53 from Mdm2 and sensitizes cancer cells to chemotherapy," J. Biol. Chem. 277: 5484-5489 (2002)). Nevertheless, how Akt disables apoptosis and its contribution to clinical drug resistance is unclear.

Although Akt has been recognized as both a likely determinant of the outcome of cancer therapy and a promising therapeutic target (Hsu, J., et al., Blood, 2001, 98(9): 2853-5; Borlado, L.R., et al., FASEB J, 2000, 14(7): 895-903; Brennan, P., et al., Oncogene, 2002, 21(8): 1263-1271; Carey, G.B. and D.W. Scott, J. Immunol, 2001, 166(3): 1618-1626; Hideshima, T., et al., Oncogene, 2001, 20(42): 5991-6000; Hyun, T., et al., Blood, 2000, 96(10): 3560-3568; Roymans, D. and H. Slegers, Eur J Biochem, 2001, 268(3): 487-498), pharmacological inhibitors of Akt are not yet available. However, one of its downstream effectors, mTOR (mammalian target of rapamycin), can be targeted with rapamycin or its ester CCI-779, which is in clinical trials against a variety of malignancies. It is important to target this pathway for cancer therapies, and to use a valid model to test these therapies and the nature of their action.

One of the difficulties in identifying determinants of drug cytotoxicity of tumor cells *in vivo* is the limited availability of appropriate material. Human tumor lines grown as xenographs are unphysiological, and the wide variation between human individuals, not to mention treatment protocols, makes clinical studies difficult. Consequently, oncologists are forced to perform correlative studies with a limited number of highly dissimilar samples, often leading to confusing and unhelpful results.

Animal models provide a useful alternative to studies in humans and to human tumor cell lines grown as xenographs, as large numbers of genetically-identical individuals can be treated with identical regimens. Moreover, the ability to introduce into the mouse germline mutations which affect oncogenesis increases the power of mouse models.

### SUMMARY OF THE INVENTION

The present invention is as defined by the appended claims.

The disclosure provides the components of in vivo and in vitro systems and methods which use them to study the effects of altered expression of a gene activity, such as the human akt, bcl-2, eIF4E or PTEN activities, on the descendants of stem cells that have been engineered to give rise to hematopoietic tumorigenic or tumor cells, such as lymphomas, with a high frequency. The present disclosure provides vectors, cells and mammals, and methods which in part depend on such products, useful for understanding tumorigenesis and its treatments, and in particular, for identifying and studying inhibitors and activators associated with tumor cell growth and growth inhibition, cell death through apoptotic pathways, and changes in apoptotic pathway components that affect drug sensitivity and resistance in tumorigenic cells. Methods for identifying molecular targets for drug screening, identifying interacting gene activities, for identifying therapeutic treatments and for identifying candidates for new therapeutic treatments are provided.

### BRIEF DESCRIPTION OF THE DRAWING

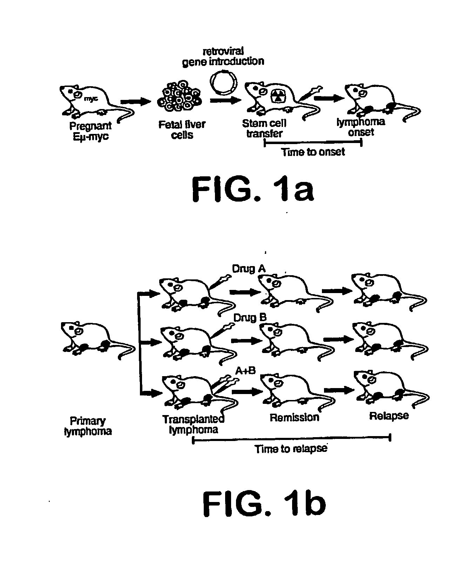

Figures 1A and 1B together are a diagram of the scheme used to produce mice that can be used to study the effect of a gene introduced into lyraphomas. In Figure 1A, hematopoietic stem cells (HSCs) were harvested from the fetal livers of *Eµ-myc* transgenic mice and retrovirally transduced with a retrovirus, MSCV, encoding the gene of interest and GFP. Non-transgenic mice were reconstituted with these HSCs following lethal irradiation. The time between transplantation and manifestation of palpable lymphomas represents the 'time to onset'. In Figure 1B, tumor cells were harvested and injected into multiple recipient mice. Upon tumor formation, the mice were treated with single agents or combinations and evaluated for their immediate response, time to relapse and survival.

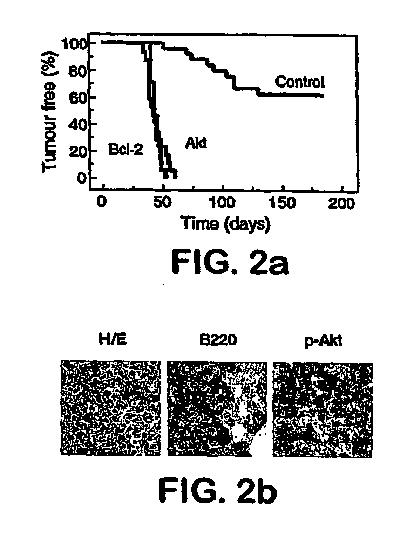

Figure 2A is a graph showing time to tumor onset following reconstitution of the hematopoietic system in non-transgenic C57BL/6 mice with fetal liver cells derived from *Eµ-myc* transgenic embryos transduced with either empty parent vector pMSCV-IRES-GFP (n=40, black), pMSCV-Bcl-2-IRES-GFP (n=18, blue), or pMSCV-Akt-IRES-GFP (n=18, green). A kinase-dead mutant Akt (K179A) did not accelerate lymphomagenesis, and Akt did not cause tumor formation in non-transgenic HSCs (data not shown). Figure 2B shows representative micrographs of *Eµ-myc*/*Akt* lymphoma sections stained with haematoxylin and eosin (H/E), the rat antibody against mouse CD45R/B220-clone RA3-6B2 (B220), and the antibody against phosphorylated-Akt (p-Akt).

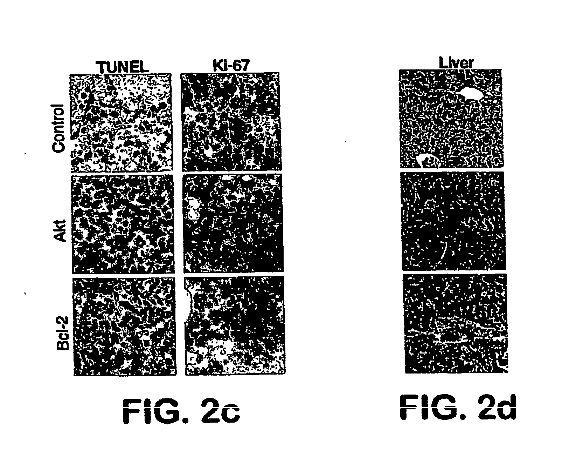

Figure 2C shows TUNEL (left) and Ki-67 (right) staining of control, Akt and Bcl-2 lymphoma sections. TUNEL was used to assess apoptosis and Ki-67 was used to assess proliferation in cells of the indicated genotypes. Extensive staining (in brown) with antibodies to the Ki67 antigen in all sections indicating a high rate of proliferation. In contrast, TUNNEL analysis showed much less DNA fragmentation or apoptosis occurring in Akt or Bcl-2 lymphoma cells relative to control lymphoma cells. Figure 2D shows photomicrographs of H/E stained liver sections showing perivascular infiltration in control tumors and parenchymal invasion in Akt and Bcl-2 tumors.

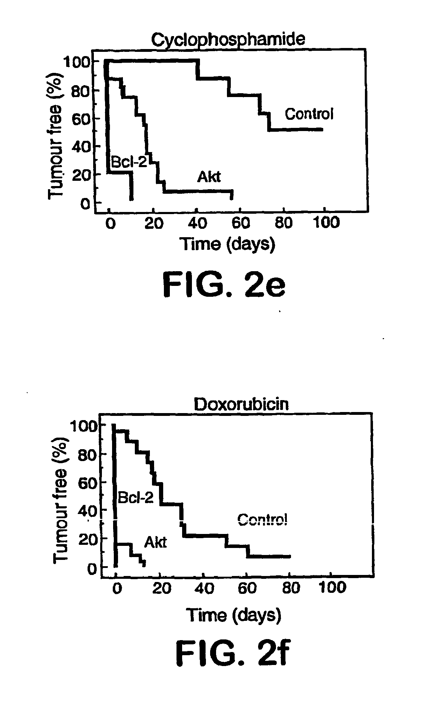

Figures 2E and F are Kaplan-Meier curves showing the tumor free survival following treatment with cyclophosphamide (Cytoxan, CTX) (E) or DXR (F). Mice bearing either Arf -/- control lymphomas (black; DXR, n=19; CTX, n=13), Akt lymphomas (green; DXR, n=25; CTX, n=16) or Bcl-2 lymphomas (blue; DXR, n=6; CTX, n=5) were treated and monitored for time to relapse.

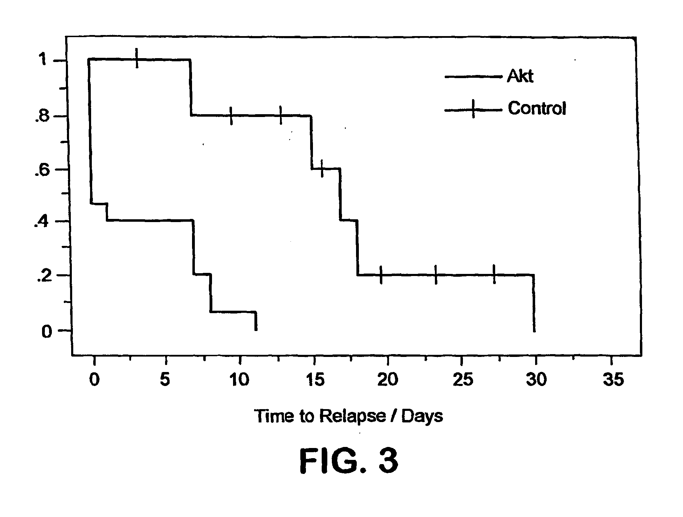

Figure 3 is a Kalpan-Meier curve showing the time of tumor free survival of mice bearing Akt tumors and Arf -/- control tumors following treatment with adriamycin at 10 mg/kg (i.p.) n=20, p=0.0009 (Mantel-Cox test).

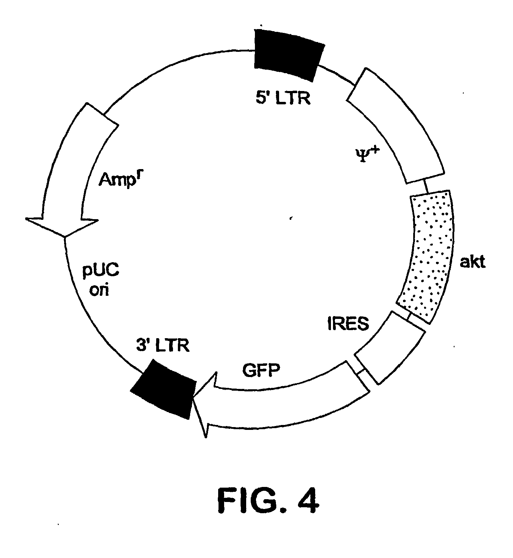

Figure 4 is a map of the vector pMSCV-Akt-IRES-GFP.

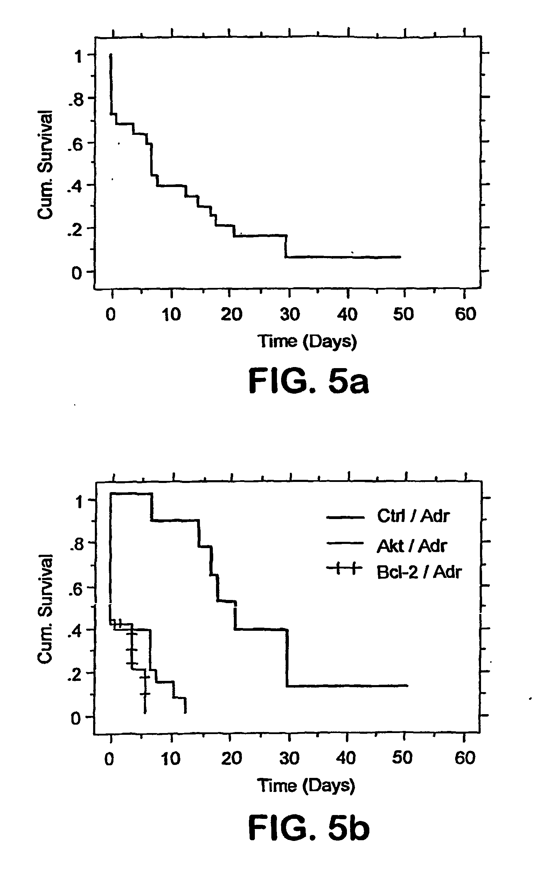

Figure 5A is a Kaplan-Meier curve showing the proportion of surviving mice for each day after adriamycin treatment. Secondary tumors were produced and treated in mice as described in Example 1. Shown are pooled data from the first eight mice in each treatment group, receiving adriamycin treatment. The tumors are from more than five separate primary tumors. Figure 5B is a Kaplan-Meier curve showing the differences in response to adriamycin treatment according to the genotype of the tumor in the treated mice. Data from mice were stratified according to the genotypes.

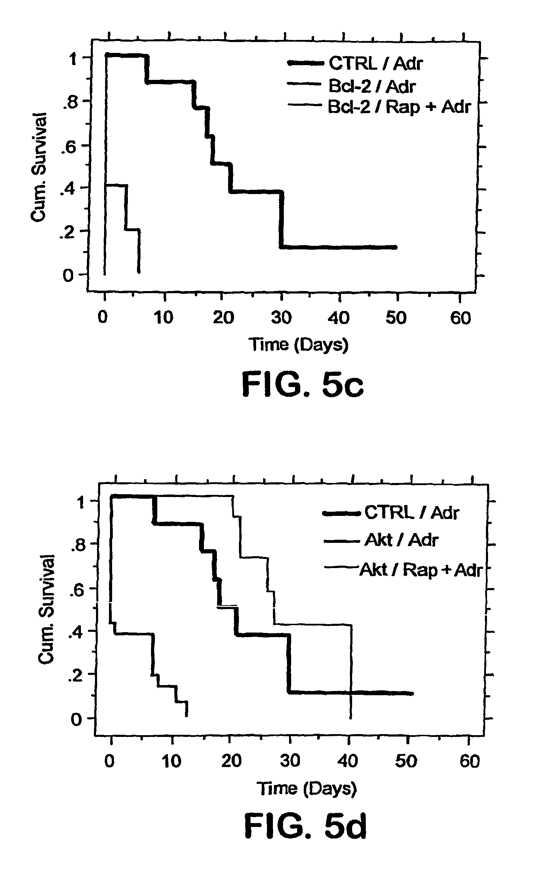

Figure 5C is a Kaplan-Meier curve showing the response of tumors arising from cells transduced with pMSCV-Bcl-2-IRES-GFP to adriamycin or a combination of adriamycin and rapamycin administered to mice bearing the tumors. Figure 5D is a Kaplan-Meier curve showing the response of tumors arising from cells transduced with pMSCV-Akt-IRES-GFP to adriamycin or to a combination of adriamycin and rapamycin administered to mice bearing the tumors.

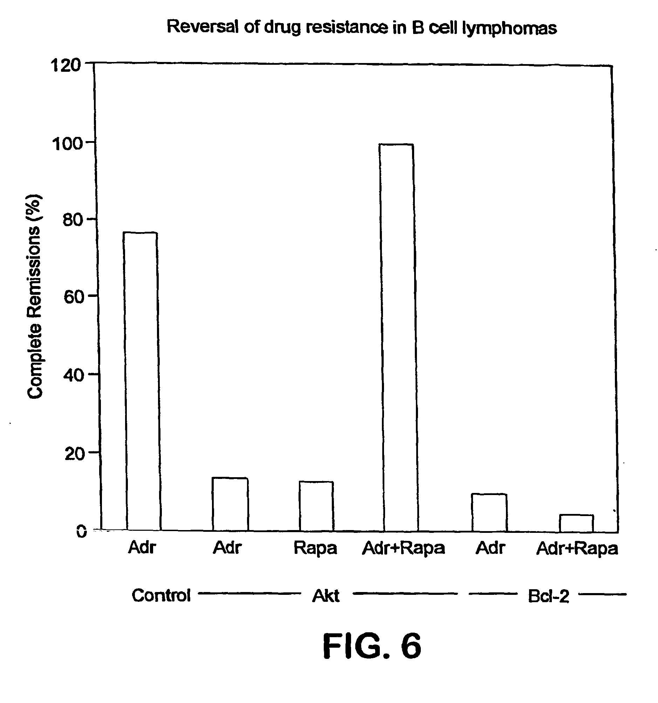

Figure 6 is a bar graph showing the proportion of mice with secondary lymphomas (produced as in Example 1) that were observed to have a complete remission following treatment with adriamycin, rapamycin, or a combination of the two.

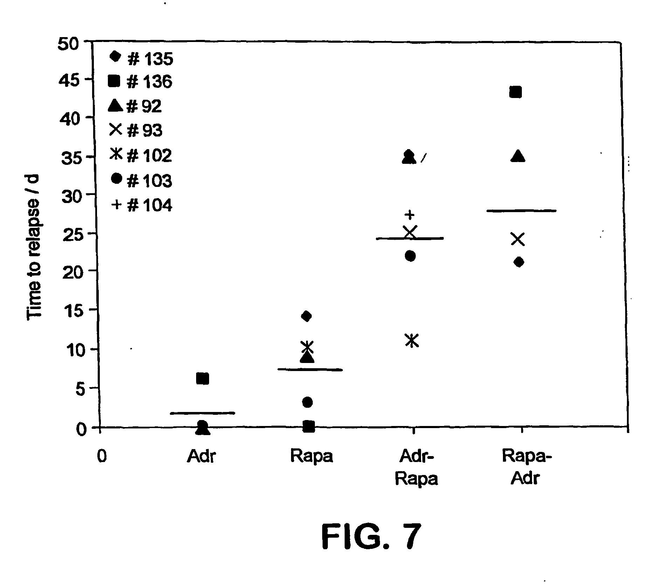

Figure 7 is a graph showing the time until relapse (in days) for mice treated for secondary lymphomas produced from seven independently arising primary tumors (#135, #136, #92, #93, #102, #103, #104). Treatment regimen was adriamycin alone (10 mg/kg. by i.p. injection) or rapamycin alone (4 mg/kg by i.p. injection), or adriamycin-rapamycin in combination (adr-rapa: adriamycin and rapamycin given on day 1, days 2-5 rapamycin alone) or rapamycin-adriamycin in combination (rapa-adr: d1 rapamycin, d2 both drugs by separate injection, d3-5 rapamycin).

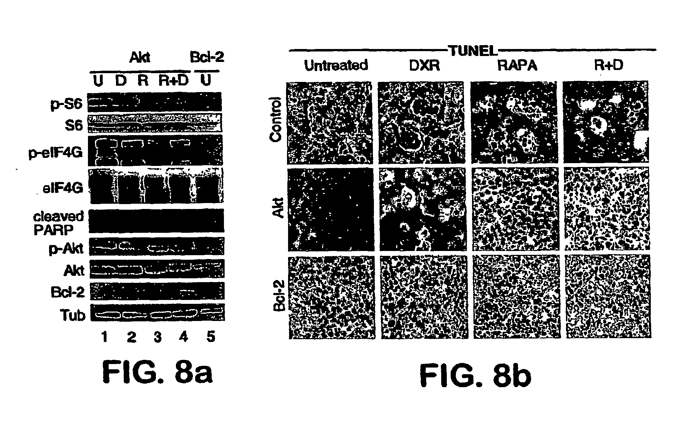

Figure 8A shows immunoblots of lysates for phosphorylated and total ribosomal S6 protein (p-S6 and S6), phosphorylated and total eIF4G (p-eIF4G and eIF4G), Akt (p-Akt and Akt), Bcl-2, and a-tubulin (Tub) as a loading control. Animals harbouring control, Akt or Bcl-2 lymphomas were either left untreated (U) or treated with DXR (A), RAP (R) or DXR and RAP (R+D) and lymphoma cells were harvested 7 hours later. Figure 8B shows lymphoma sections derived from untreated or treated animals as listed above stained with TUNEL (brown) to identify apoptotic cells in the indicated genotypes.

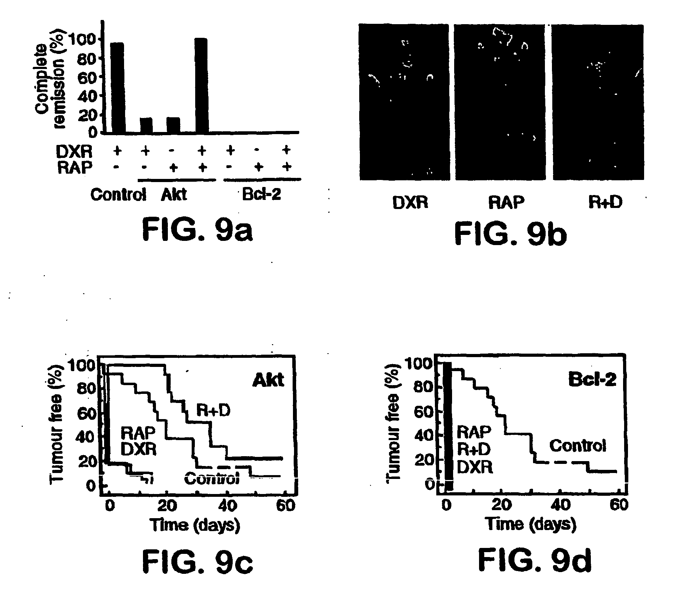

Figure 9A is a bar graph representing the percentage of mice bearing control (n = 19), Akt (n = 51) or Bcl-2 (n = 18) expressing lymphomas that achieved a complete remission (CR) following the indicated treatment. The number of mice in each treatment group ranged from 6 to 25. Figure 9B is whole body fluorescence imaging of GFP expression of a 'matched group' of mice carrying identical Akt tumors 21 days after the indicated treatment. Figure 9C is a Kaplan-Meier curve showing the tumor free survival in Akt lymphomas following treatment with DXR (red, n = 25), RAP (blue line, n = 12) and RAP+DXR (green, R+D, n = 14). Figure 9D is a Kaplan-Meier curve showing (D) Bcl-2 lymphomas following treatment with DXR (n = 6; red), RAP (n = 6, blue), RAP+DXR (R+D, n = 6, green). The tumor free survival of the control tumors following DXR or CTX treatment is shown for comparison (dotted line).

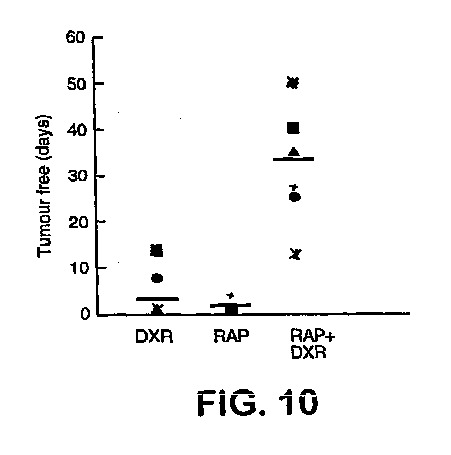

Figure 10 is a graph showing the time until relapse (in days) for mice treated for secondary lymphomas produced from eight independently arising primary tumors. Mice bearing matched tumors were generated by injecting 1x10⁶ tumor cells from the same primary tumor into three recipient animals. Eight 'matched groups' of three mice received DXR, RAP, or DXR + RAP. Mice were monitored twice weekly by palpation and blood smears. The graph indicates the individual tumor free survival times and the arithmetic mean; a paired t-test analysis comparing either combination to the single agents confirmed statistical significance (for DXR versus RAP+DXR, p = 0.0002; RAP versus RAP+DXR, p < 0.0001; while DXR versus RAP, p = 0.7).

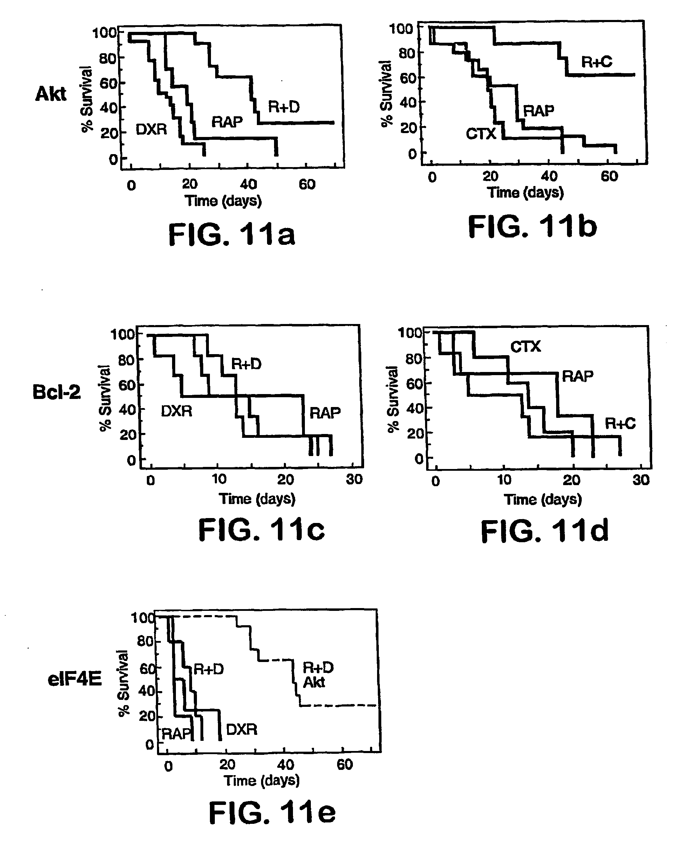

Figure 11 is a series of Kaplan-Meier curves showing the overall survival of mice treated with rapamycin alone or in combination with conventional chemotherapy. Kaplan-Meier analyses of time to death (pre-terminal condition) following treatment of *Eµ-myc*/Akt (top panels), *Eµ-myc* /bcl-2 (middle panels) and *E*µ-*myc*/eIF4E lymphomas (bottom panel) with RAP (blue), DXR (in A, C and E, red) or CTX (in B and D, red), or the combinations (green) RAP+DXR (A, C and E) and CTX+RAP (B and D). The combination of RAP with DXR or CTX resulted in a prolonged survival in mice bearing Akt expressing tumors (DXR trial, n= 51; CTX trial, n=36), but did not extend the survival of mice carrying Bcl-2 or eIF4E expressing tumors (Bcl-2 tumors: CTX trial, n=17; DXR trial, n=18; eIF4E tumors, DXR trial, n=17). The survival of Akt tumor bearing mice treated with RAP+DXR is indicated as a dotted line in E.

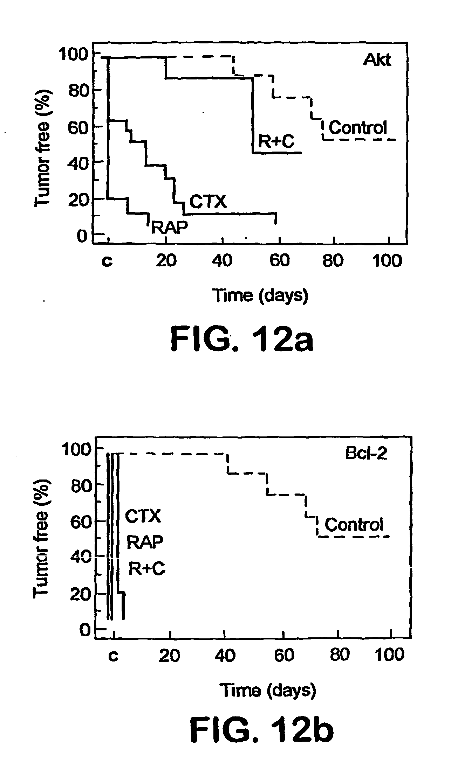

Figure 12A is a Kaplan-Meier curve showing tumor free survival in Akt tumor bearing mice following treatment with CTX (n=16, red), RAP (n=12, blue), or CTX+RAP (C+R, n = 8). Figure 12B is a Kaplan-Meier curve showing tumor free survival in Bcl-2 tumor bearing mice following treatment with CTX (n=6, red), RAP (n=6, blue) and CTX+RAP (C+R, n=4, green). The tumor free survival of the control tumors following CTX treatment is indicated in the black dotted line.

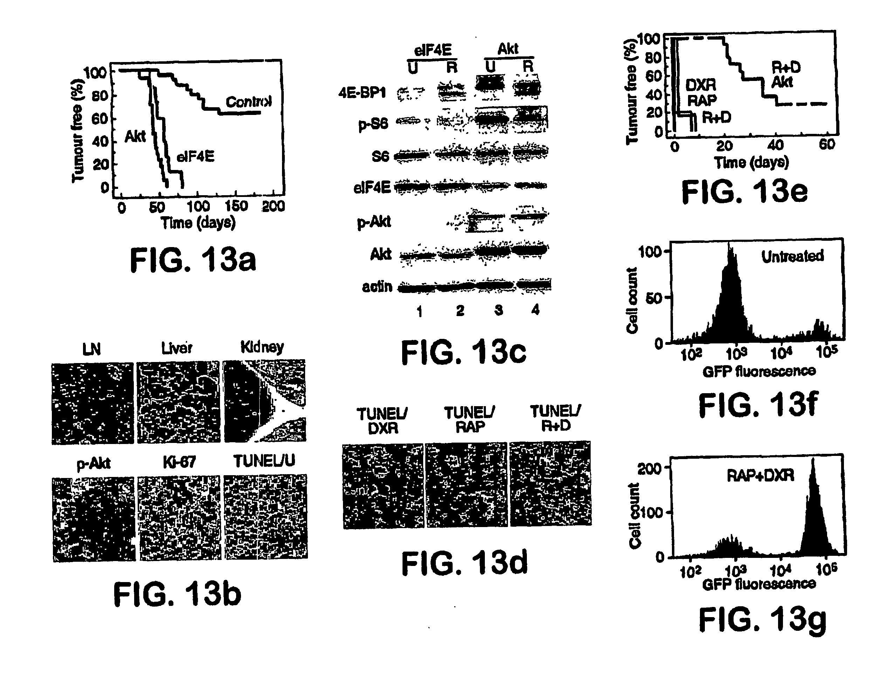

Figure 13A is a Kaplan-Meier curve showing the time to tumor development, following hematopoietic reconstitution with *Eµ-myc* transgenic HSCs expressing eIF4E (n =15, p<0.0001 for eIF4E versus pMSCV-control). The tumor onset curves for HSCs transduced with control pMSCV and Akt are shown for comparison. eIF4E did not induce tumors in a non-transgenic background within the observation period (> 125 days). Figure 13B shows representative micrographs of eIF4E lymphoma sections: top panel, haematoxylin and eosin (H/E) staining of the indicated organs; lower panel, immunohistochemical stains for phosphorylated Akt, Ki-67 and TUNEL in untreated tumors. Figure 13C shows immunoblotting of lysates derived from eIF4E and Akt lymphomas harvested from untreated (U) animals or six hours after rapamycin (R) administration using antibodies against the indicated protein ("p" denotes an antibody specific for a phospho-protein). Untreated Akt tumors (lane 3) show an increase in the phosphorylated, slower migrating forms of 4E-BP1. Figure 13D shows TUNEL staining (brown) to assess apoptosis in tumor sections harvested 6-8 hours following the indicated therapy. Figure 13E is a Kaplan-Meier curve showing tumor free survival of mice bearing eIF4E lymphomas following treatment with DXR (n= 5, red line), RAP (n= 6, blue), RAP+DXR (n= 6, green line). Data from mice bearing Akt lymphomas treated with RAP+DXR are shown for comparison (dotted line). Figures 13F and G show an in vivo competition assay. An Akt lymphoma was transduced with MSCV-eIF4E-GFP such that the resulting population contained only ∼2-5% GFP-positive cells and then transplanted into multiple recipient mice. Upon lymphoma manifestation, the animals were left untreated (E) or treated (F) with rapamycin/DXR therapy. Lymphoma cells were isolated 48 hours later and subjected to flow cytometry to determine the fraction of eIF4E-positive cells (high GFP fluorescence).

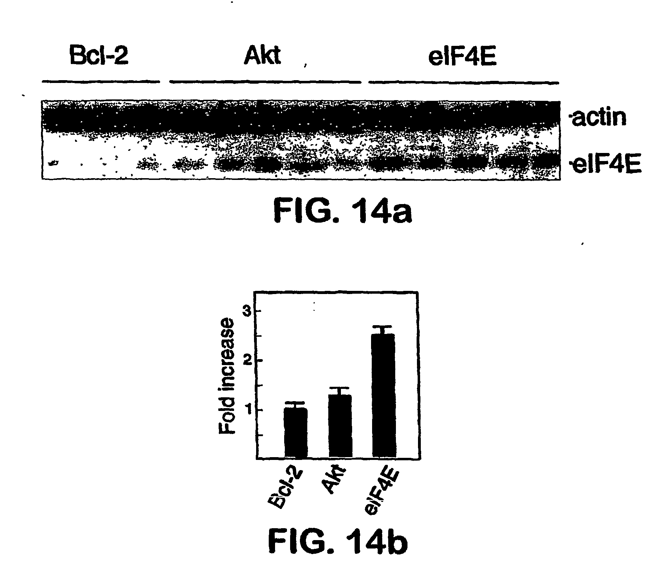

Figure 14 shows the quantification of eIF4E expression in lysates derived from Bcl-2 (n=3), Akt (n=5) and eIF4E (n=5) tumors. (A) Lysates were probed with antibodies against eIF4E and b-actin as loading control and I¹²⁵ labeled Protein A. Radioactivity was quantitated on Western blots by phosphorimaging. (B) Graphic representation of eIF4E expression relative to Akt and Bcl-2 tumors. The presented data are the combined averages of four independent probings of the tumor set with error bars denoting the standard deviation. Bcl-2 (n=3), Akt (n=5), eIF-4E (n=5).

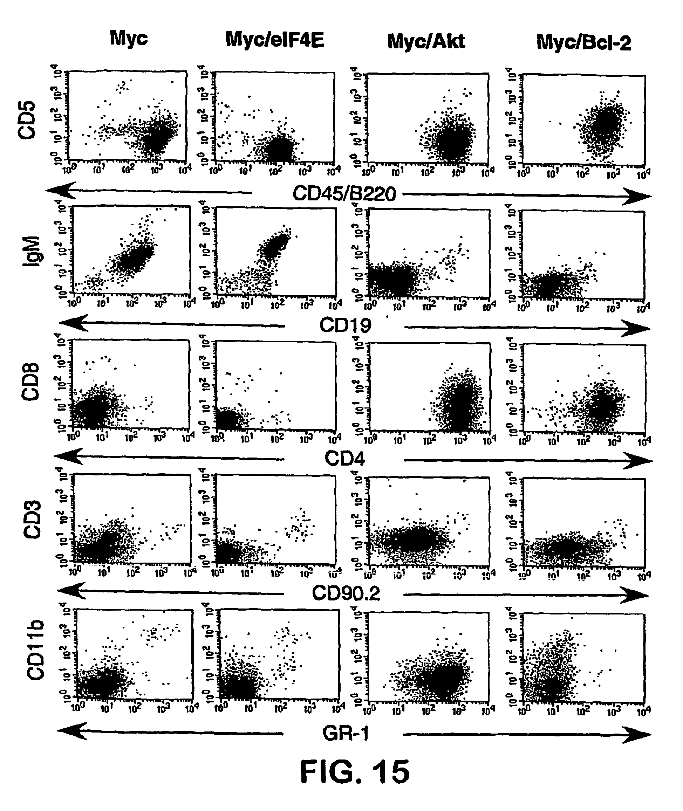

Figure 15 shows representative data from flow cytometric immunophenotyping of control (*Eµ-myc*), Bcl-2 (*Eµ-myc*/bcl-2), Akt *(Eµ-myc*/akt) and eIF4E *(Eµ-myc*/eIF4E) tumors. Tumor cells were gated on the basis of forward scatter and side scatter and GFP positivity. Both Akt and Bcl-2 tumors did not express B-cell antigens other than CD45R (B220) but were positive for CD4, whereas the control and eIF4E tumors had a mature B-cell marker profile. At least three tumors of each genotype were analyzed.

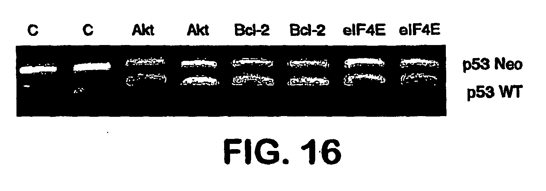

Figure 16 shows allele-specific PCR to detect the wild-type (p53 WT) and mutant allele (p53 Neo) in tumors derived from *Eµ-myc* /p53+/- HSCs. Shown are representative results from two tumors per group, these are either control (C), Akt, Bcl-2 or eIF4E tumors. All control tumors showed loss of heterozygosity (LOH) (8/8), while none of the Akt (0/5), Bcl-2 (0/5) or eIF4E (0/3) tumors underwent LOH.

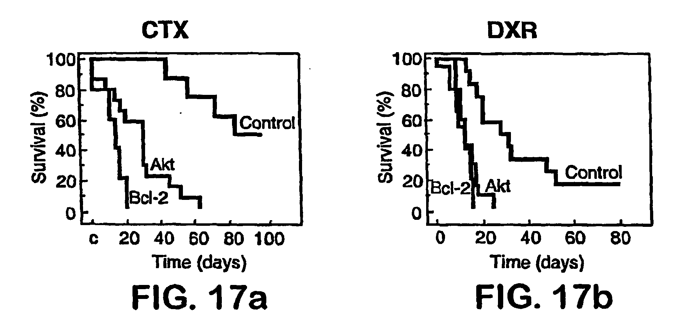

Figure 17A and 17B are two Kaplan-Meier curves showing the survival time following treatment with CTX (A) and DXR (B). Mice bearing either control lymphomas (black; DXR, n=19; CTX, n=13), Akt lymphomas (green; DXR, n=25; CTX, n=16) or Bcl-2 lymphomas (blue; DXR, n=6; CTX, n=5) were monitored for time to terminal disease or death.

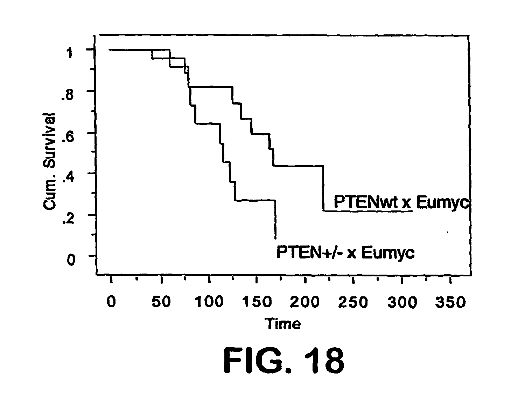

Figure 18 is a Kaplan-Meier curve showing the survival time from birth to tumor development in mice from a cross of a transgenic *Eµ-myc* mouse to a PTEN +/- mouse. The control group is *Eµ-myc* /PTEN +/+ (green line, n=24) and the experimental group *Eµ-myc*/PTEN +/- (green line, n=12). PTEN heterozygosity causes a significant acceleration and increased penetrance in lymphoma development in vivo (p<0.05).

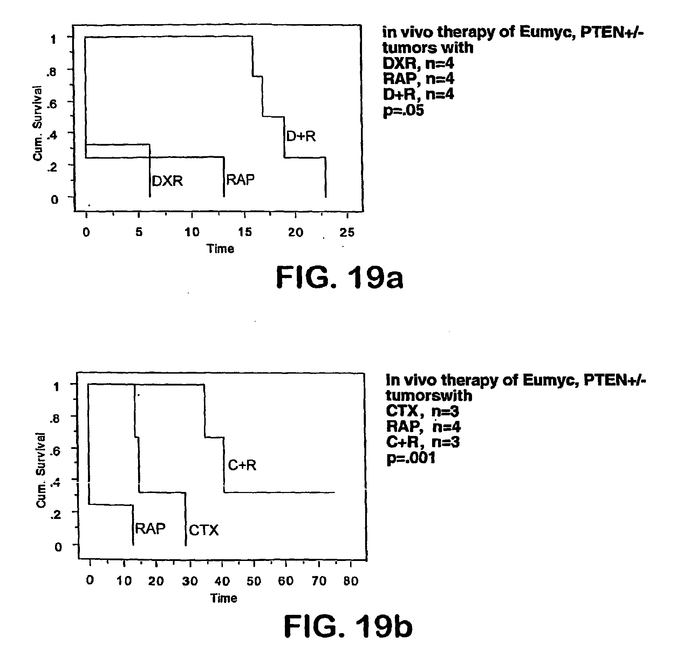

Figure 19A is a Kaplan-Meier curve showing tumor free survival of C57BL/6 mice bearing *Eµ-myc*/PTEN +/- tumors following treatment with doxorubicin (DXR), rapamycin (RAP), and the combination of both (RAP+DXR). Tumor free survival implies the complete absence of detectable disease, i.e. no frank leukemia and no palpable tumors, a relapse denotes the recurrence of either. Figure 19B is a Kaplan-Meier curve showing an equivalent analysis for treatment with cyclophosphamide (CTX), rapamycin (RAP) and the combination (RAP+CTX).

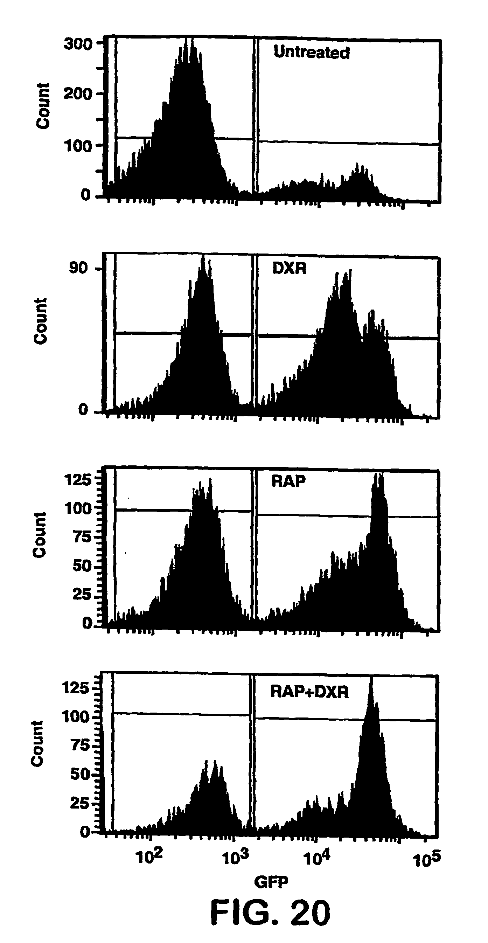

Figure 20 shows results from flow cytometry in *Eµ-myc*/PTEN +/- tumor cells infected with eIF4E 48 hours after in vivo therapy. The x-axis indicates the GFP fluorescence, denoting tumor cells that are productively infected with MSCV-eIF4E-IRES-GFP. In vivo therapy was a single injection of either doxorubicin (DXR) or rapamycin (RAP) or a combination of both (RAP + DXR). An observed increase in GFP-positive cells is noted in all treated tumors as compared to the untreated control.

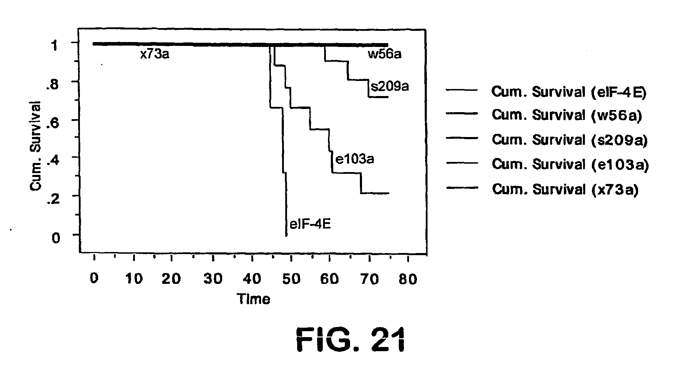

Figure 21 is a Kaplan-Meier curve showing time to tumor development in various eIF4E mutants. Hematopoietic stem cells were harvested from the fetuses of a pregnant *Eµ-myc* mouse (ED13-15) and infected in vitro with MSCV-IRES-GFP constructs expressing different mutants of eIF4E, including E103A, S209A, W56A, and W73A.

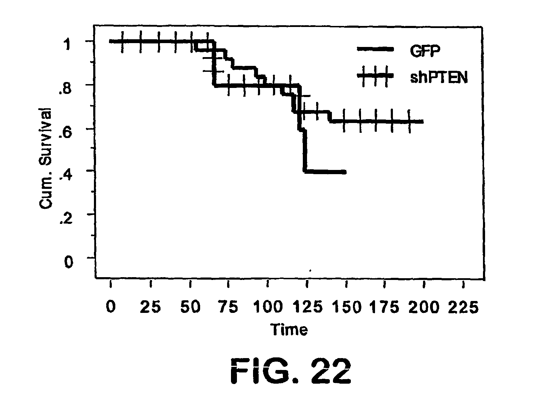

Figure 22 is a Kaplan-Meier curve showing the time to tumor development in *Eµ*-*myc*/short hairpin PTEN (shPTEN) mice following reconstitution of lethally irradiated mice with HSCs from the fetal livers of infected with a short hairpin against PTEN (MSCV-shPTEN-IRES-GFP). The control group are equivalent HSCs infected with a vector encoding only GFP (controls; n=25, shPTEN, n=5).

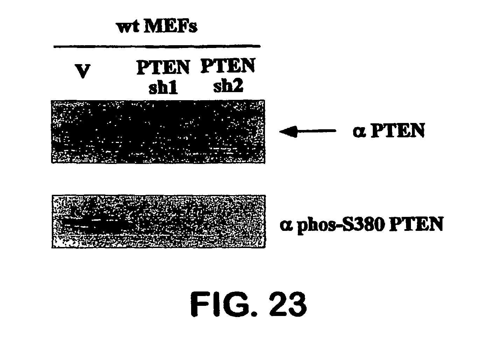

Figure 23 is an immunoblot showing down-regulation of PTEN expression in the presence of PTEN short hairpins. Wild type Mouse Embryo Fibroblasts (wt MEFs) containing a control vector (V) or a short hairpin against two mPTEN sequences (PTEN sh1 and PTEN sh2) were collected and analyzed for the expression of endogenous total and phosphorylated PTEN by Western blotting.

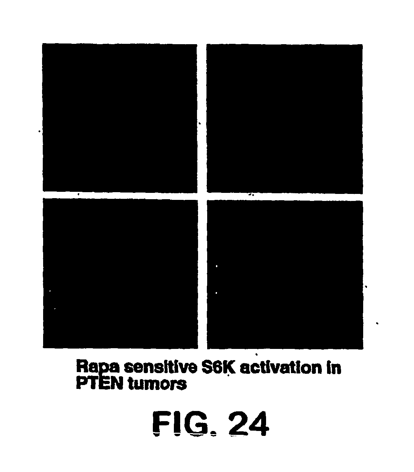

Figure 24 shows immunofluorescent staining of *Eµ-myc*/PTEN +/- tumor cells incubated with 10 nM rapamycin and incubated with antibodies to S6-kinase. DAPI (4', 6-diamidino-2-phenylindole dihydrochloride) was added with the antibody to stain all cells (blue stain on top panels). Untreated cells fluoresced brightly (left), but cells treated with rapamycin treatment (right) showed a marked inhibition of S6-kinase (red staining).

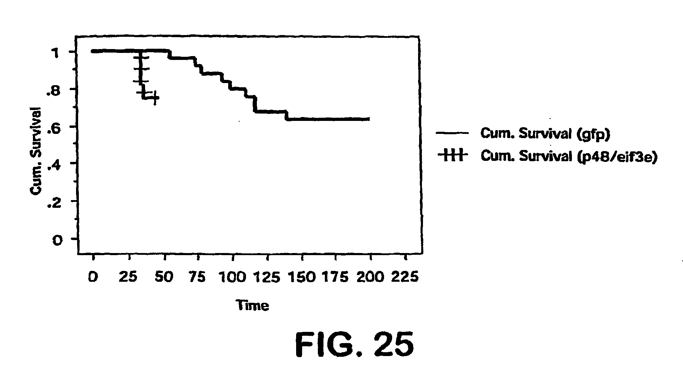

Figure 25 is a Kaplan-Meier curve showing tumor free survival of E*µ-myc*/p48/eIF3E +/-mice following reconstitution of lethally irradiated mice with HSCs from the fetal livers of infected with p48 fragment of eIF3E (MSCV-eIF3E-IRES-GFP). The control group are equivalent HSCs infected with a vector encoding only GFP (controls; n=25, eIF3E; n=16).

### DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, cell and cancer biology, virology, immunology, microbiology, genetics and protein and nucleic acid chemistry described herein are those well known and commonly used in the art.

The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2003); Harlow and Lane Antibodies: A Laboratory Manual Cold Spring Harbor Laboratory Press, Cold Spring Harbor, N.Y. (1990); Coffin et al. Retroviruses, Cold Spring Harbor Laboratory Press; Cold Spring Harbor, N.Y. (1997); Bast et al., Cancer Medicine, 5th ed., Frei, Emil, editors, BC Decker Inc., Hamilton, Canada (2000); Lodish et al., Molecular Cell Biology, 4th ed., W. H. Freeman & Co., New York (2000); Griffiths et al., Introduction to Genetic Analysis, 7th ed., W. H. Freeman & Co., New York (1999); Gilbert et al., Developmental Biology, 6th ed., Sinauer Associates, Inc., Sunderland, MA (2000); and Cooper, The Cell - A Molecular Approach, 2nd ed., Sinauer Associates, Inc., Sunderland, MA (2000).

The following terms, unless otherwise indicated, shall be understood to have the following meanings:

As used herein, the term "hematopoietic tumorigenic [or tumor] cell" refers to a cell which is or which may become committed to a hematopoietic lineage and which exhibits an altered growth phenotype. The term encompasses cells which are associated with hematopoietic malignancy (i.e., lymphomas, leukemias) as well as with pre-malignant conditions such as lymphoproliferative and myeloproliferative disorders.

As used herein, the term "hematopoietic stem cell" refers to any multipotent cell which can produce committed and/or differentiated cells of the hematopoietic lineage. Hematopoietic stem cells include myeloid (including erythroid, granulocytic and megakaryocytic) and lymphoid precursor cells and cells not yet committed to either lineage.

As used herein, the term "well defined genotype" refers to a cell of the invention characterized by comprising a defined combination of genetic lesions. A preferred cell with a "well defined genotype" is one having, e.g., activated myc in combination with one or more activated akt, bcl-2 and eIF4E activities (or repressed PTEN or 4E-BP activity). Such lesions are typically introduced into a cell, individually or in any desired combination, by means of DNA (vector)-mediated gene transfer.

As used herein, the term "nucleic acid or "nucleic acid molecule" refers to a polymeric form of nucleotides of at least 10 bases in length. The term includes DNA molecules (e.g., cDNA or genomic or synthetic DNA) and RNA molecules (e.g., mRNA or synthetic RNA molecules), as well as analogs of DNA or RNA containing non-natural nucleotide analogs, non-native internucleotide bonds, or both. The nucleic acid can be in any topological conformation. For instance, the nucleic acid can be single-stranded, double-stranded, triple-stranded, quadruplexed, partially double-stranded, branched, hairpinned, circular, or in a padlocked conformation.

As used herein, the term "mutation" refers to any change in the nucleic acid or amino acid sequence of a gene product. The term "mutated" when applied to nucleic acid sequences means that nucleotides in a nucleic acid sequence may be inserted, deleted, rearranged or otherwise changed compared to a reference nucleic acid sequence. A single alteration may be made at a locus (a point mutation) or multiple nucleotides may be inserted, deleted or changed at a single locus. In addition, one or more alterations may be made at any number of loci within a nucleic acid sequence. A nucleic acid sequence may be mutated by any method known in the art including but not limited to in vivo and in vitro mutagenesis techniques such as "error-prone PCR" (a process for performing PCR under conditions where the copying fidelity of the DNA polymerase is low, such that a high rate of point mutations is obtained along the entire length of the PCR product. See, e.g., Leung, D. W., et al., Technique, 1, pp. 11-15 (1989) and Caldwell, R. C. & Joyce G. F., PCR Methods Applic., 2, pp. 28-33 (1992)); and "oligonucleotide-directed mutagenesis" (a process which enables the generation of site-specific mutations in any cloned DNA segment of interest. See, e.g., Reidhaar-Olson, J. F. & Sauer, R. T., et al., Science, 241, pp. 53-57 (1988)).

As used herein, the phrase "degenerate variant" of a reference nucleic acid sequence encompasses nucleic acid sequences that can be translated, according to the standard genetic code, to provide an amino acid sequence identical to that translated from the reference nucleic acid sequence.

The nucleic acids (also referred to as polynucleotides) of this invention may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The term "vector" as used herein is intended to refer to a nucleic acid molecule capable of transporting another nucleic acid to which it has been linked. One type of vector is a "plasmid", which refers to a circular double stranded DNA loop into which additional DNA segments may be ligated. Other vectors include cosmids, bacterial artificial chromosomes (BAC) and yeast artificial chromosomes (YAC). Another preferred type of vector is a viral vector, wherein additional DNA segments may be ligated into a viral genome that is usually modified to delete one or more viral genes. Certain vectors are capable of autonomous replication in a host cell into which they are introduced (e.g., vectors having an origin of replication which functions in the host cell). Other vectors can be integrated stably into the genome of a host cell upon introduction into the host cell, and are thereby replicated along with the host genome. Preferred viral vectors include retroviral and lentiviral vectors. Moreover, certain preferred vectors are capable of directing the expression of nucleic acid sequences to which they are operatively linked. Such vectors are referred to herein as "recombinant expression vectors" (or simply, "expression vectors").

"Operatively linked" expression control sequences refers to a linkage in which the expression control sequence is contiguous with the gene of interest to control the gene of interest, as well as expression control sequences that act in *trans* or at a distance to control the gene of interest.

The term "expression control sequence" as used herein refers to polynucleotide sequences which are necessary to affect the expression of sequences (e.g., coding sequences) to which they are operatively linked. Expression control sequences are sequences which control the transcription, post-transcriptional events and translation of nucleic acid sequences. Expression control sequences include transcription initiation sequences (i.e., promoter and enhancer sequences) which control the position and rate of transcription initiation. In addition, expression control sequences include appropriate transcription termination sequences, efficient RNA processing signals such as splicing and polyadenylation signals; sequences that stabilize nuclear and/or cytoplasmic mRNA; sequences that enhance translation efficiency (e.g., ribosome binding sites); sequences that enhance protein stability; and when desired, sequences that enhance protein secretion. The nature of such control sequences differs depending upon the host organism. The term "control sequences" is intended to include, at a minimum, all components whose presence is essential for expression, and can also include additional components whose presence is advantageous, for example, leader sequences, stabilization sequences and fusion partner sequences.

The term "overexpression" as used herein with reference to a nucleic acid sequence refers to a higher level of transcription and/or translation of a nucleic acid or protein product encoded by a nucleic acid sequence in a cell. Overexpression is most commonly accomplished by operative linkage of nucleic acid sequences to a strong promoter/enhancer sequence which stimulates transcription in the target host cell. Overexpression can be non-regulated (i.e., a constitutive "on" signal) or regulated (i.e., the "on" signal is induced or repressed by another signal or molecule within the cell).

The term "recombinant host cell" (or simply "host cell"), as used herein, is intended to refers to a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell or cell line grown in culture or may be a cell which resides in a living tissue or organism.

The term "peptide" as used herein refers to a short polypeptide, e.g., one that is typically less than about 50 amino acids long and more typically less than about 30 amino acids long. The term as used herein encompasses analogs and mimetics that mimic structural and thus biological function.

The term "polypeptide" encompasses both naturally-occurring and non-naturally-occurring proteins, and fragments, mutants, derivatives and analogs thereof. A polypeptide may be monomeric or polymeric. Further, a polypeptide may comprise a number of different domains each of which has one or more distinct activities.

As used herein, the term "molecule" means any compound, including, but not limited to, a small molecule, peptide, protein, sugar, nucleotide, nucleic acid, lipid, etc., and such a compound can be natural or synthetic.

As used herein, the term "gene X activity" refers to the biological-activity encoded by the "gene X" (e.g., akt, bcl-2, eIF4E, PTEN, myc or the like), and to that activity encoded by a homologous gene or gene product from another organism or by an unrelated gene or gene product from the same or different organism. Biological activity may be measured by monitoring any physiological function or attribute of "gene X". It may include enzymatic assays, binding assays, immunoassays, structural and chemical assays (e.g., which measure modifications such as phosphorylation status, conformational changes) and the like.

As used herein, the term "altered activity" refers to a change in the level of a gene and/or gene product with respect to any one of its measurable activities in a cell (e.g., the function which it performs and the way in which it does so, including chemical or structural differences and/or differences in binding or association with other factors). An altered activity may be effected by one or more structural changes to the nucleic acid or polypeptide sequence, a chemical modification, an altered association with itself or another cellular component or an altered subcellular localization. An altered activity may thus be "activated" or "increased", "repressed" or "decreased", or may remain "normal" (albeit at altered levels). An "activated" or "increased" activity refers, e.g., to overexpression of an encoding nucleic acid, an altered structure (e.g., primary amino acid changes or post-transcriptional modifications such as phosphorylation) which causes higher levels of activity, a modification which causes higher levels of activity through association with other molecules in the cell (e.g., attachment of a targeting domain) and the like. Likewise, "repressed" or "decreased" activity refers, e.g., to decreased expression of an encoding nucleic acid (e.g., through antisense or inhibitory RNAi approaches), an altered structure (e.g., primary amino acid changes or post-transcriptional modifications such as phosphorylation) which causes reduced levels of activity, a modification which causes reduced levels of activity through association with other molecules in the cell (e.g., binding proteins which inhibit activity or sequestration) and the like.

As used herein, the term "hematopoietic stem cell" refers to a precursor cell capable of reconstituting part or all of the hematopoietic system of a mammal, such stem cells further characterized by their ability to self-renew and to differentiate into cells of the myeloid and/or lymphoid lineages.

An "agent" as used herein can be, for example, a pharmaceutical or chemical with known physiological effects, such as pharmaceuticals that have been used in chemotherapy for cancer. Agents include chemotherapeutic agents. Chemotherapeutic agents inhibit proliferation of tumor cells, and generally interfere with DNA replication or cellular metabolism. Chemotherapeutic agents may or may not have been characterized for their target of action in cells.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Tenns (Parker, S., Ed., McGraw-Hill, San Francisco (1985)), incorporated herein by reference).

"Sensitivity" and "resistance" as used herein refer to the extent to which tumor growth is inhibited by a treatment. If tumor growth is inhibited by the treatment to a statistically significant degree, compared to a control, the tumor is sensitive to the treatment. If tumor growth is not inhibited by the treatment to a statistically significant degree, compared to a control, the tumor is resistant to the treatment. Tumor sensitivity/resistance can also be relative. For example, a tumor previously sensitive to a treatment can become resistant in a relapsed form, where growth of the relapsed form is not inhibited to the same extent as that seen by the equivalent treatment of the primary tumor.

Unless otherwise defined, all technical and scientific terms used herein have the same meaning as commonly understood by one of ordinary skill in the art to which this invention pertains. Exemplary methods and materials are described below, although methods and materials similar or equivalent to those described herein can also be used in the practice of the present invention and will be apparent to those of skill in the art.

Throughout this specification and embodiments, the word "comprise" or variations such as "comprises" or "comprising", will be understood to imply the inclusion of a stated integer or group of integers but not the exclusion of any other integer or group of integers. Where the singular terms vector, cell, mammal and mouse are used in the embodiments, the plural meaning is also included, as a single vector, cell, mammal or mouse would not usually be used in isolation.

In case of conflict, the present specification, including definitions, will control. The materials, methods, and examples are illustrative only and not intended to be limiting.

A description of certain preferred embodiments of the invention follows.

### Expressing Akt Signaling Pathway Genes in Hematopoietic Stem Cells

We report an animal model for testing genes involved in apoptotic signaling pathways -- such as the *akt* gene (encoding the Akt serine-threonine kinase; also known as PKB); *bcl-2* gene (encoding b-cell lymphoma-2 gene, overexpressed in human follicular lymphoma), *eIF4E* gene (encoding eukaryotic translation initiation factor 4E); and the *PTEN* gene (encoding a phosphatase with homology to tensin and which is a negative regulator of Akt kinase) for their effects on anti-cancer treatments, the development of drug resistance and treatments to encourage drug sensitivity of tumorigenic cells, especially hematopoietic tumorigenic cells.

The function of Akt was investigated by cloning the gene encoding human Aktl and expressing an active form of Akt in hematopoietic stem cells. Specifically, Akt 1 was inserted into a murine stem cell virus (MSCV) vector carrying a gene encoding green-fluorescent protein (GFP) which was used to infect hematopoietic stem cells that were harvested from the bone marrow or fetal livers of *Eµ-myc* mice (see, e.g., US 6,583,333). These stem cells were used to reconstitute the hematopoietic system of lethally irradiated mice (Figure 1A). Thus, the entire hematopoietic compartment in these mice is derived from the genetically modified *Eµ-myc* stem cells, while the other tissues of the animal are wild type. In these mice the *myc* transgene is activated during B cell development and causes lymphoma formation. This approach allowed us to study the impact of the akt transgene on tumorigenesis (see Example 1). The tumors expressing the transgene are easily identified by their GFP expression and can be harvested, split, and introduced into multiple non-transgenic syngeneic mice to form secondary lymphomas (Figure 1B). This increases the numbers of animals for treatment studies (Schmitt, C.A. and S.W. Lowe, Blood Cells Mol Dis, (2001), 27(1): 206-216, Schmitt, C.A., C.T. Rosenthal, and S.W. Lowe, Nat Med, (2000), 6(9): 1029-1035). Using this approach, we found that Akt dramatically accelerates the onset of lymphomas arising in pMSCV-Akt-transduced hematopoietic stem cells taken from E*µ*-*myc* transgenic mice (Example 1; Figure 2A). In part, this may be due to its anti-apoptotic activity, as Akt expression appears to obviate the need for these tumors to lose the pro-apoptotic p53 gene.

While conferring a selective advantage *in vivo,* Akt producing B-cells could not be grown in tissue culture under any of a variety of conditions tested, underlining the necessity for a suitable *in vivo* model. Expression of Akt changes the metabolic requirements of tumor cells. Unlike Bcl-2, which inhibits apoptosis at the mitochondrial level and confers a general survival advantage, Akt appears to be responsible for maintaining mitochondrial membrane potential through increasing glycolysis. See, e.g., Plas, D.R. et al., J. Biol. Chem. 276(15):12041-12048, (2001). While Bcl-2 expressing cells grow well under any culture conditions, Akt expressing cells are very sensitive to culture conditions. Based on Plas *et al.* and other reports, we tried a variety of conditions (different concentrations of glucose, 3% oxygen, addition of IL3, IL6, IL7 or nonessential amino acids to the media), but none of these modifications of the media allowed us to grow Akt expressing B-lymphoma cells in culture. A pure population of Akt-expressing cells isolated from a tumor derived by using the stem cell infection and adoptive transfer approach also does not grow in culture at all. In a mixed population of pMSCV-Akt-IRES-GFP infected and uninfected B lymphoma cells, the proportion of GFP positive cells dropped from between 25% to 35%, depending on the experiment, to undetectable, within 48 hours. This dependence on environmental cues underlines the necessity to study Akt in an *in vivo* model, as presumably, the only way to keep Akt expressing cells viable in culture is through accumulation of other genetic defects (loss of p53, etc.) which, while allowing growth in culture, clearly interfere with their use as research tools to study the PI3K/Akt pathway and pharmacological interference with it.

To determine how Akt signaling influences the response of tumors to chemotherapy *in vivo,* the effects of a constitutively activated Akt mutant (Andjelkovic, M., et al., "Role oftranslocation in the activation and function of protein kinase B," J. Biol. Chem. 272: 31515-31524 (1997)) to the anti-apoptotic regulator Bcl-2 in the *Eµ-myc* model of B-cell lymphoma (Adams, J. M., et al. "The c-myc oncogene driven by immunoglobulin enhancers induces lymphoid malignancy in transgenic mice," Nature 318: 533-538 (1985)), were compared, as described in Example 2. In brief, *Eµ-myc* hematopoietic stem cells isolated from fetal livers were transduced with Akt or Bcl-2 expressing retroviruses, transplanted into lethally irradiated mice and the chimeric recipients were monitored for lymphoma onset and pathology (Figure 1 depicts the method and Figure 2A charts the results).

Resulting lymphomas were each transplanted into several wild type animals, where they produced malignancies that are pathologically indistinguishable from the original disease (see Example 1 and Schmitt, C. A. and Lowe, S. W, "Bcl-2 mediates chemoresistance in matched pairs of primary Eµ-myc lymphomas in vivo," Blood Cells Mol. Dis. 27: 206-216 (2001)). As described in Example 2, these animals were then treated with a chemotherapeutic regime and monitored for time to relapse ("tumor free survival") and overall survival by lymph node palpation and blood analysis. This approach allows a rapid analysis of genotype-response relationships in a natural setting using methods that parallel human clinical trials.

Further, in treatment studies, it has been found that, compared to control tumors (Arf-null tumors used as controls; the response patterns of these tumors are uniquely homogeneous with regard to p53 status), the akt transgene confers a high degree of resistance to cyclophosphamide (Figure 2E) and adriamycin (Figure 3). Both are agents central to the conventional therapy of lymphoma. Specifically, the Akt producing tumors relapse significantly earlier than do the control tumors. This translates into a dramatic reduction in survival, especially for the cyclophosphamide treatment, which is curative for many of the control tumors but none of the Akt tumors.

### VECTORS

### Akt-vectors

In one aspect, the invention comprises vectors carrying a gene, constructed for high level expression of the gene, starting from vectors based on murine stem cell virus (MSCV). The vectors can comprise an akt gene as described in the prior art, or other akt genes to be isolated as natural variants or produced by manipulation in the laboratory. The Akt protein produced in cells into which the vector is introduced can have varying levels of phosphorylation activity, according to the Akt gene chosen to be constructed into the vector. A particular vector described herein is pMSCV-Akt-IRES-GFP, which encodes human Akt and which has the advantage of carrying a linked marker gene encoding green fluorescent protein.

One important feature of a vector that can be used to introduce a gene into recipient cells is a high frequency of integration into the recipient cell genome. Another desirable feature of such a vector is a high level of constitutive gene expression for the inserted gene of interest. Vectors that are derived from the murine stem cell virus (MSCV) have been developed for studying gene function in hematopoietic, embryonic stem cells and embryonal carcinoma cells. Following insertion of a gene into the vector, the vector is transfected into a packaging cell line to allow for replication of the nucleic acid and packaging of viruses which are unable to replicate in cells not engineered to produce retroviral proteins. Cell lines for producing amphotropic virus, as well as cell lines for producing ecotropic virus, are available. For vectors carrying a human oncogene to be expressed in cells, a cell line to produce ecotropic virus is desirable for safety concerns. Construction and propagation of useful MSCV vectors is described, for example, in Hawley, R.G. et al., Proc. Natl. Acad. Sci. USA 93:10297-10302 (1996) and in Chen, L. et al., Blood 1:83-92 (1998), and in the catalog and technical information from Clontech/BD Biosciences, which supplies pMSCVneo and related pMSCV vectors, along with packaging cell lines.

pMSCV-based vectors carrying a human Akt oncogene are referred to here as pMSCV-Akt vectors. A pMSCV-Akt vector comprises, at a minimum, a vector comprising 5' and 3' LTRs of the murine stem cell virus (MSCV), an akt gene with expression driven by the LTR from MSCV, and ψ⁺ for expressing the extended viral packaging signal (see Example 1 and Figure 4). The akt gene can be a human gene. The akt gene can be wild-type (normal), a mutant or variant found in nature, or an artificially mutated or modified gene. The gene can encode a gene product differing from that of a wildtype gene product, such as an inactive gene product or a gene product conferring a dominant negative phenotype. The pMSCV-Akt-IRES-GFP vector, cells transduced with it, and animals bearing such cells are discussed below as illustrations of a more general pMSCV-gene vector, in which any gene of interest can be inserted, for example, an oncogene or a gene suspected to play a role in apoptosis, or in tumor maintenance (see Example 1).

A pMSCV-based vector, pMSCV-Akt-IRES-GFP, was constructed to provide high level expression of the human akt gene in hematopoietic stem cells and their progeny that undergo differentiation. Although the combination of IRES and GFP in the vector allows for the convenient separation, quantitation and *in vivo* detection of cells transduced with the MSCV-Akt-IRES-GFP vector, either IRES or GFP or both would not be essential elements in a pMSCV-Akt vector. Such a pMSCV-Akt vector lacking IRES, GFP or both, could be used, for example, in experiments that do not require physically locating the transfected cells. For example, one could use a pMSCV-Akt vector in a study in which one would test the effectiveness of an agent to inhibit the development of lymphomas, by introducing into two populations of mice a pMSCV-Akt vector, treating one population with a putative anti-lymphoma agent, but not treating, or sham-treating, the other population, and following the time until lymphomas were detected.

In one embodiment, the invention provides vectors comprising akt sequences, preferably (but not necessarily) linked to one or more expression control sequences which will regulate its expression in a host cell. In one preferred embodiment of the akt expression vector, akt activity is activated by cellular localization of the catalytic domain to the plasma membrane of the cell in which it is produced. One known way for accomplishing plasma membrane localization is through physical association of the Akt polypeptide with a myristoylation/palmitylation motif which targets the Akt polypeptide to the plasma membrane. Preferably, the myristoylation/palmitylation motif is engineered at the N-terminus of the Akt polypeptide by linkage of appropriate nucleic acid sequences and expression of the hybrid sequences in the selected host cell. It will be understood that akt activation by cellular localization can be accomplished by any means. In another preferred embodiment, plasma membrane localization of akt kinase activity remains subject to physiological regulation. Further, localization of Akt to the plasma membrane may be inducible, e.g., through an inducible association with a motif, such as a myristoylation/palmitylation motif, which mediates plasma membrane localization.

In certain preferred embodiments, the invention provides a vector capable of being introduced into a stem cell comprising a nucleic acid sequence which expresses akt activity upon expression in a hematopoietic cell. Preferably, the vector causes the cell in which it is expressed to produce an altered akt activity, i.e., which is different in phenotype (e.g., having altered activity) and/or having increased or decreased levels of "normal" akt activity relative to a control cell lacking the expressed nucleic acid sequence.

Preferably, an akt vector of the invention comprises nucleic acid sequences derived from mammalian sequences, and more preferably, from human sequences. The akt nucleic acid sequences may be wild-type, variant, derivative (e.g., fused to other domains or chemically altered) or mutated sequences. For example, in certain preferred embodiments, the encoded Akt polypeptide has altered akt biological activity. In certain embodiments, for example, it is preferred that the akt have reduced or eliminated kinase activity. Overexpression of such an akt mutant will help modulate the akt signaling pathway and is expected to facilitate the ability of a tumor cell to regain sensitivity to a drug or other agent to which it has become chemoresistant through an akt-, bcl-2-, eIF4E-mediated signaling pathway.

Also provided by the invention are vectors which are capable of introducing a nucleic acid sequence into stem cells or tumorigenic cells and which encode sequence specific inhibitors of akt activity. Preferably, the vectors encode a shRNA (short hairpin RNA) which mediates inhibition of akt signaling in a stem cell, such as a hematopoietic stem cell, or in a tumor or tumorigenic cell, especially of a hematopoietic lineage. Such vectors will be useful for making cells and mammals in which akt signaling is modulated according to the methods of the invention.

### bcl-2-, eIF4E- and PTEN vectors

In other embodiments, the invention provides a vector capable of being introduced into a stem cell comprising a nucleic acid sequence which expresses akt, bcl-2, eIF4E or PTEN activity upon expression in a hematopoietic cell. Preferably, the vector causes the cell in which it is expressed to produce an altered akt, bcl-2, eIF4E or PTEN activity, i.e., which is different in phenotype (e.g., having altered activity) and/or having increased or decreased levels of otherwise "normal" activity relative to a control cell lacking the expressed nucleic acid sequence.

The invention further provides vectors comprising bcl-2 nucleic acid sequences which can be introduced into stem cells or tumorigenic cells and from which bcl-2 sequences can be expressed. Preferably, a bcl-2 vector of the invention comprises nucleic acid sequences derived from mammalian sequences, and more preferably, from human or mouse sequences. The bcl-2 nucleic acid sequences may be wild-type, variant, derivative (e.g., fused to other domains or chemically altered) or mutated sequences. For example, in certain preferred embodiments, the encoded bcl-2 polypeptide has altered biological activity. In certain embodiments, for example, it is preferred that the bcl-2 have reduced or eliminated activity. Overexpression of such a bcl-2 mutant will help modulate the akt-/bcl-2-/eIF4E signaling pathway and is expected to facilitate the ability of a tumor cell to regain sensitivity to a drug or other agent to which it has become chemoresistant through an akt-, bcl-2-, eIF4E-mediated signaling pathway. A particular preferred vector described herein is pMSCV-bcl-2-IRES-GFP, which encodes a human bcl-2 and which has the advantage of carrying a linked marker gene encoding green fluorescent protein, as described above for akt vectors.

Also provided by the invention are vectors which are capable of introducing a nucleic acid sequence into stem cells or tumorigenic cells and which encode modulators (activators or inhibitors) of bcl-2 activity. Preferred modulators include sequence specific inhibitors of bcl-2. Preferably, the vectors encode a shRNA (short hairpin RNA) which mediates inhibition of bcl-2 signaling in a stem cell, such as a hematopoietic stem cell, or in a tumor or tumorigenic cell, especially of a hematopoietic lineage. Such vectors will be useful for making cells and mammals in which the akt-/bcl-2-/eIF4E signaling pathway is modulated according to the methods of the invention.

The invention further provides vectors comprising eIF4E nucleic acid sequences. Vectors can comprise an eIF4E gene as described in the prior art, or other eIF4E genes to be isolated as natural variants or produced by manipulation in the laboratory. The eIF4E protein produced in cells into which the vector is introduced can have varying levels of binding activity to other components of the multimolecular complexes involved in translation initiation, resulting in varying rates or levels of efficiency of translation of mRNAs, according to the eIF4E gene chosen to be constructed into the vector. A particular preferred vector described herein is pMSCV-eIF4E-IRES-GFP, which encodes a murine eIF4E and which has the advantage of carrying a linked marker gene encoding green fluorescent protein, as described above for akt vectors.

Another preferred eIF4E vector of the invention comprises a mutation at a phosphorylation site (S209A) which is the target of a kinase (MNK-1 or -2) in the MAP kinase pathway (through MEK). Phosphorylation of S209 was shown previously not to be inhibited by rapamycin. In contrast, as shown herein, a stem cell transduced with an eIF4E vector comprising the S209A mutation, when introduced into mouse hematopoietic stem cells engineered to become tumor cells, produces a mouse which exhibits a significantly longer time to onset of tumor formation than does a mouse expressing a corresponding non-mutated eIF4E vector.

Also provided by the invention are vectors which are capable of introducing a nucleic acid sequence into stem cells or tumorigenic cells and which encode modulators (activators or inhibitors) of eIF4E activity. Preferred modulators include sequence specific inhibitors of eIF4E activity. Preferably, the vectors encode a shRNA (short hairpin RNA) which mediates inhibition of eIF4E signaling in a stem cell, such as a hematopoietic stem cell, or in a tumor or tumorigenic cell, especially of a hematopoietic lineage. Other preferred vectors encode 4E-BP activity (4E-BP1, 2 and/or 3) which bind to eIF4E and thereby inhibit its activity. Such eIF4E vectors will be useful for making cells and mammals in which the akt-/bcl-2-/eIF4E signaling pathway is modulated according to the methods of the invention.

The invention further provides vectors comprising PTEN nucleic acid sequences which can be introduced into stem cells or tumorigenic cells and from which PTEN sequences can be expressed. Inhibition of PTEN introduced into mouse hematopoietic stem cells engineered to become tumor cells produced a mouse which exhibited a significantly reduced time to onset of tumor formation than did a control mouse (see Example 12). Thus, overexpression of PTEN is expected to result in decreased akt signaling and increased sensitivity to rapamycin in combination with chemotherapeutic agents.

Also provided by the invention are vectors which are capable of introducing a nucleic acid sequence into stem cells or tumorigenic cells and which encode modulators (activators or inhibitors) of PTEN activity. Preferred modulators include sequence specific inhibitors of PTEN activity. Preferably, the vectors encode a shRNA (short hairpin RNA) which mediates inhibition of PTEN and thus akt signaling in a stem cell, such as a hematopoietic stem cell, or in a tumor or tumorigenic cell, especially of a hematopoietic lineage. Two exemplary vectors which encode shRNAs that mediate PTEN inhibition are described herein (see also SEQ ID NOS:1 and 2). Such PTEN vectors will be useful for making cells and mammals in which akt-/bcl-2-/eIF4E signaling is modulated according to the methods of the invention.

In general, a vector of the invention must be capable of being introduced into a stem cell, preferably a mammalian stem cell, and more preferably, a hematopoietic stem cell. The vector may be introduced by any of numerous methods known in the art for introducing nucleic acids into eukaryotic cells. See, e.g., Ausubel et al., Current Protocols (*supra*)*.* In a preferred embodiment, the vector of the invention is a viral vector capable of transducing a mammalian stem cell, and preferably, a hematopoietic stem cell. A variety of viral vectors are envisioned and the invention is not limited to the viral vector type as long as the vector can successfully infect or transduce a stem cell with the desired activity.

Preferred viral vectors are retroviral based vectors. One preferred retroviral vector is derived from MMLV. Another preferred retroviral vector (used in the Examples of the invention set forth herein) is the murine stem cell virus, "MSCV" (see, e.g., McCurrach and Lowe, Methods Cell Biol. 66:197-227 (2001) for exemplary retroviral methods; see also Coffin et al., Retroviruses, supra (1997)). Another preferred viral vector is a lentiviral based vector that can transduce or infect a stem cell, and preferably, a hematopoietic stem cell. Many retroviral and lentiviral vector systems and packaging cell lines are known in the art and available.

Other preferred vectors of the invention comprise a nucleic acid sequence which expresses akt, bcl-2, eIF4E or PTEN activity upon expression in a hematopoietic cell wherein part or all of the nucleic acid sequence encoding such activity can be deleted or excised from surrounding sequences even after it has integrated into the host genome. Any of a number of sequences which mediate excision from DNA upon expression of a cognate recombinase and corresponding recombinase activities may be used to induce such excisions or deletions, e.g., crelox recombinase/lox-P sites; Flp recombinase/Frt sites; and pfiC31/att sites. A nucleic acid sequence encoding akt, bcl-2, eIF4E or PTEN activity is flanked by sequence motifs that mediate deletion of internal sequences upon expression of the cognate recombinase activity. Preferably, the recombinase is operatively linked to an inducible promoter. Thus, for example, the invention provides, e.g., a creestrogen receptor fusion protein induced by tamoxifen to delete the transduced gene so that induction causes recombinase-mediated excision of akt, bcl-2, eIF4E or PTEN-encoding sequences from the genome. Alternatively, a vector containing the loxP sites flanking the gene may be introduced into stem cells harboring a transgene that conditionally expresses cre recombinase. In either case, upon tumor formation, the introduced gene may be deleted. As discussed *supra*, reduction or elimination of akt, bcl-2 or eIF4E biological activity will increase the sensitivity of a tumor cell to certain therapeutic treatments to which a tumor cell has acquired resistance through activation of an akt-, bcl-2-, and/or eIF4E-mediated signaling pathway.

Various embodiments of the invention involve expressing akt signaling pathway components having altered (e.g., increased or decreased) activity. As shown using the mouse models for tumorigenesis described herein, increasing activity of akt-, bcl-2 or eIF4E or decreasing activity of the akt inhibitor PTEN leads to activated akt signaling and increased chemoresistance, whereas decreasing activity of akt-, bcl-2 or eIF4E or increasing activity of the akt inhibitor PTEN leads to decreased akt signaling and increased chemosensitivity. Thus, for purposes of producing mammalian models for studying genes (e.g., as potential therapeutic targets), genetic alterations and for screening potential drug candidates which can alter chemosensitivity or resistance, it will be desirable to modulate one or more of each of these activities in either direction. Modulation of other components of the pathway that interact with Akt-, Bcl-2, eIF4E (e.g., 4E-BP) or PTEN is also envisioned. Accordingly, also encompassed by the present invention are nucleic acid molecules which can effectively modulate (e.g., inhibit) expression of a target gene activity (i.e., akt-, bcl-2, eIF4E or PTEN) in a cell or a mammal of the invention. Preferably, shRNAs (short hairpin RNAs) are used as sequence specific expression inhibitors using "RNAi" techniques which are well known in the art. See, e.g., Scherer and Rossi, Nature Biotechnology 21:1457-65 (2003) for a review on sequence-specific mRNA knockdown of using antisense oligonucleotides, ribozymes, DNAzymes, RNAi and siRNAs.

Certain preferred vectors of the invention comprise nucleic acid sequences encoding the activity of interest (akt, bcl-2, eIF4E or PTEN) and further comprise one or more additional gene(s) capable of expression. The additional gene(s) may encode one or more of a variety of functions. For example, it may encode a selectable marker gene whose presence and/or whose expression co-localizes with the presence and/or expression of the activity of interest (akt, bcl-2, eIF4E or PTEN). The additional gene may encode a detectable marker gene such as a fluorescent marker (e.g., GFP, luciferase, color-shifted variants and the like). In some preferred embodiments, the additional gene may encode an oncogenic activity which contributes to the ability of a cell harboring the vector to become transformed, e.g., into cells having malignant or pre-malignant phenotypes, especially of the hematopoietic system. As described herein, a mammal that receives cells transduced with such vectors will develop tumors, especially hematopoietic tumors. In certain preferred embodiments, the oncogene is an activated *myc* gene, such as one whose expression is operatively linked to a strong viral promoter and/or enhancer. One such preferred vector comprises the Eµ- *myc* gene, which is derived from a cellular *myc* gene activated by insertion of an Ig enhancer. Of course, any other means for activating expression of the *myc* gene may be used in the vectors (and cells and mammals) of the invention.

In other preferred embodiments, the additional gene may encode a sequence specific expression inhibitor, such as one described above. Preferred inhibitors encode one or more shRNAs (short hairpin RNAs) or other inhibitory nucleic acid which, upon expression in a stem cell or primary hematopoietic tumor cell (e.g., a lymphoma), modulate the activity of the akt-/bcl-2-/eIF4E signaling pathway thereby affecting tumorigenesis, apoptotic cell death and chemosensitivity and resistance.

In other embodiments, the additional gene may be transcriptionally linked to nucleic acid sequences encoding the activity of interest (akt, bcl-2, eIF4E or PTEN). If so, it is preferred that they be translated from a downstream internal ribosome entry site (IRES) rather than being expressed as part of a hybrid fusion protein. Thus, certain preferred vectors of the invention comprise a nucleic acid sequence encoding an activity of interest (e.g., akt, bcl-2, eIF4E or PTEN) linked to a sequence encoding IRES-gene 2 sequence. Preferred retroviral vectors of the invention thus include pMSCV-akt-IRES-gene 2, pMSCV-bcl-2-IRES-gene 2, pMSCV-eIF4E-IRES-gene 2, and pMSCV-akt-PTEN-gene 2. It is also possible to switch the order of gene 2 such that it is transcribed upstream from (5' to) an IRES-nucleic acid sequence encoding the activity of interest (akt, bcl-2, eIF4E or PTEN). Moreover, there is no reason to limit such constructs to bicistronic messages. Tri- or multi-cistronic mRNAs may be constructed using several IRES sequences in tandem and are encompassed in the present invention.

### CELLS

Vectors of the invention can be introduced into cells, for example, by viral transduction, and a high frequency of integration of the vector can be achieved. The cells can be cells of a mammal, preferably a rodent such as a rat, more preferably a mouse, and even more preferably, a human cell. The cells for study can be chosen for a genotype that facilitates study of pathways involved in apoptosis and resistance to chemotherapeutic agents. For example, the cells can be *Eµ-myc* mouse cells, and, in a particular embodiment, are *Eµ-myc* mouse hematopoietic stem cells. The cells into which the vectors have been introduced are another aspect of the invention.

The invention provides cells transduced with a pMSCV-Akt vector (e.g., pMSCV-Akt-IRES-GFP). Transduction procedures are well known in the art and have been described. A transduction procedure is illustrated in several of the Examples. Transduced cells integrate the pMSCV-Akt-IRES-GFP vector into the cell genome at a high frequency, and stably express the akt gene. Protein extracted from the transduced cells can be analyzed for Akt, for example, by western blot, using antibodies specific to Akt, as illustrated (e.g., Example 2, Example 7, and Examples 9-11). Transduction and integration of the pMSCV-Akt-IRES-GFP vector allows for tracking of the progeny of the transduced cells generations after the originally transduced cells, by detection of the GFP marker. Other markers which can mark and trace cells and their progeny are also encompassed as preferred markers of the invention.

Several of the Examples illustrate the use of cells of a specific genotype, derived from mice having an activated *myc* oncogene (e.g., under the control of the Eµ IgH enhancer). The *myc* gene can be one as described in Harris, A.W., J. Exp. Med. 167:353-371 (1988) or the allele described by Langdon, W.Y. et al., Cell 47:11-18 (1986), for example. The *myc* gene can also be a naturally-occurring gene, either cellular or viral, a natural variant or an artificially altered variant of *myc.* Any other oncogene that can accomplish a similar purpose is also encompassed as a preferred additional gene of the invention.

Although the use of a pMSCV-Akt vector in mouse hematopoietic stem cells is described in detail in the Examples, any other type of mammalian cells can be infected with a pMSCV-Akt vector, if an amphotropic retrovirus packaging cell line is used to produce the virus. The resulting cells can be used in a variety of studies on the effects of Akt, for example, the effects on the downstream effectors of Akt, the effect of Akt on apoptosis, cell death, resistance to anti-proliferative treatments (for example, drugs to be used in cancer chemotherapy) and the effects of Akt on metabolism and metabolic regulation.

Cells of the invention include any cell into which a vector of the invention has been introduced. Preferably, the cells of the invention are mammalian, and more preferably, are rodent (mouse or rat), primate or human. Preferred cells of the invention are stem cells (pluripotent or multipotent), and are, more preferably, hematopoietic stem cells. Also preferred are tumor cells, especially primary tumor cells, into which a vector of the invention has been introduced. Cells of the invention are useful for producing mammals of the invention, especially by implantation of stem cells and repopulation of hematopoietic cells of the mammal (see below). Certain cells of the invention are prone to becoming hematopoietic tumor cells. Other cells of the invention are more resistant to becoming hematopoietic tumor cells. Accordingly, cells of the invention are also useful as tools in methods for studying cell growth and regulation, apoptosis, tumorigenesis, and sensitivity and resistance to chemical agents such as small molecules and other chemicals which may be useful as drugs in the treatment of diseases, disorders or conditions associated with abnormal regulation of such cellular processes.

### MAMMALS

In a further aspect of the invention, the cells described above are introduced into one or more non-human recipient mammals (e.g., mice), thereby producing trangenic mammals bearing the cells of the invention. The recipient mammal is engineered to develop hematopoietic tumorigenic or tumor cells such as lymphomas which comprise a gene that modulates the akt-, bcl-2-, eIF4E-, PTEN-, eIF3E- pathway.

Thus, the invention provides a mammal into which a vector or cell of the invention has been introduced. Preferably, the mammals of the invention are rodents (e.g., mouse or rat), primates.

We have produced and characterized an animal model for testing the effects of modulating activity of the akt gene, bcl-2 gene, eIF4E gene, PTEN gene, and eIF3E gene on various anti-cancer treatments, and on the development of drug resistance in tumor cells. Also included in the invention are methods for using the mammals of the invention, e.g., mice that develop lymphomas, and the lymphoma cells that arise in the mouse model, to elucidate the nature of drug resistance, to study the effects of gene overexpression (or inhibition) on apoptosis-related signaling pathways and on levels of other regulatory factors in the cell.

Thus, a further aspect of the invention is a mammal bearing a population of cells transduced with a pMSCV-Akt vector such as pMSCV-Akt-IRES-GFP. Mammals bearing such transduced cells can be studied, for example, for the effect of Akt in apoptosis, in development of resistance to agents that promote apoptosis and in treatments to reduce such resistance.

A particular example of mammals bearing a population of cells transduced with a pMSCV-Akt vector are mice produced by starting with C57BL/6 mice, obtaining fetal liver or bone marrow from them, culturing hematopoietic stem cells from this tissue, and transducing akt into these cells using pMSCV-Akt-IRES-GFP. The transduced cells are introduced into syngeneic or lethally irradiated C57BL/6 recipient mice, whereupon the transduced cells reseed the bone marrow, thereby producing a population of cells having a genome comprising a *myc* gene operably linked to an Eµ IgH enhancer, and further comprising an akt gene by insertion of pMSCV-Akt-IRES-GFP into the recipient mouse genome.

The mouse model described herein provides a useful model for studying apoptosis and cancer therapy, since: (i) tumor burden can be monitored externally by lymph node palpation and often by blood smears; (ii) lymphomas are detectable long before the animals die, so animals can be treated while otherwise healthy; (iii) large numbers of tumor cells can be isolated from mice undergoing therapy or treatment; (iv) therapy is performed in immunocompetent mice; and (v) lymphoma cells can be transplanted into syngeneic and/or sub-lethally irradiated mice.

### METHODS

The cells and mammals of the invention, and methods which use them, offer advantages over prior art cells, mammals and methods for studying the role of signaling pathway components which regulate oncogenesis, tumor growth, apoptosis and the development of drug resistance. In the prior art cells and methods, undefined tumors with relatively undefined and unknown genotypes were used to study factors involved in regulating apoptosis, chemosensitivity and the development of resistance to therapeutic agents. In contrast, the present invention provides model cells and mammalian systems having a well-defined starting genotype.

In another embodiment, the invention provides a method for testing a hematopoietic tumor or tumor cell for sensitivity to a particular test treatment. In certain aspects, the method involves administering a test treatment to a mammal such as a mouse which harbors a population of hematopoietic cells in which the activity of akt, bcl-2 and/or eIF4E is increased or activated relative to a control animal and/or that of PTEN and/or eIF3E is decreased or repressed relative to a control animal. The mouse has also been engineered to develop a hematopoietic tumor, wherein some or all of the tumor cells have an activated signaling pathway characteristic of increased akt, bcl-2 and/or eIF4E expression and/or decreased PTEN and/or eIF3E expression. The mouse is monitored for the time to onset of primary tumor formation (e.g., palpable tumor formation), and an increased time to tumor onset indicates sensitivity to the treatment. The invention thus further provides a method for identifying agents or treatments which increase the time to tumor onset in a mammal having a tumor or tumor cells with an activated signaling pathway characteristic of increased akt, bcl-2 and/or eIF4E expression and/or decreased PTEN and/or eIF3E expression.

The instant invention is based in part on the discovery that hematopoietic tumors of a particular genotype may become resistant to treatments to which they were originally sensitive. Chemoresistance is associated with a change in cellular signaling pathways involved in apoptosis (programmed cell death), and in particular, in akt, bcl-2 and eIF4E-mediated signaling.

Thus, in another embodiment, a mammal such as the above described mouse (i.e., having one or more hematopoietic tumors characterized by cells having increased akt, bcl-2 and/or eIF4E activity or reduced PTEN or eIF3E activity) is treated with an agent (e.g., chemotherapy) which causes remission of hematopoietic tumor cells. Those mice are then monitored for the length of time until (secondary) tumor relapse. An increased time of remission before tumor relapse indicates sensitivity of secondary tumors in the mice to the administered treatment. The invention thus further provides a method for identifying agents or treatments which increase the time to tumor relapse in a mammal having a tumor or tumor cells with an activated signaling pathway characteristic of increased akt, bcl-2 and/or eIF4E expression and/or decreased PTEN and/or eIF3E expression, the mammal having been administered a chemotherapeutic agent or treatment.

A lymphoma arising in a mouse that has received cells from *Eµ-myc* hematopoietic stem cells transduced with a pMSCV-Akt vector can be tested for sensitivity to a treatment, by administering the treatment to the mouse (designated a "test" mouse), and monitoring the mouse for a decrease in signs of the lymphoma (remission). Complete remission is a normal state of the mouse in which no lymphoma can be detected by palpation, and white blood cell counts are indistinguishable (not statistically significantly different) from those of healthy mice. Sensitivity of a lymphoma to a treatment can be measured by the length of time until relapse, that is, the time when palpable tumors are again detectable, or by the proportion of mice that survive a chosen period of time. An appropriate control mouse when comparing the effects of treatments, is for example, one which is genetically identical or very similar, and which is maintained under the same conditions as the test mouse, except that no treatment, or sham-treatment, is given.

The treatment to be tested can be one or more substances, for example, a known anti-cancer agent, such as adriamycin, rapamycin, cylophosphamide, prednisone, vincristine, cisplatin, or a radioactive source. The substance or agent can be administered preferably by intraperitoneal injection in a pharmaceutically acceptable vehicle, but also by other appropriate vehicles and routes, for example, orally, intranasally, by inhalation, intramuscular injection, hypodermic injection, intravenous injection or by surgical implantation, in topical creams, transdermal patches and the like, all optionally with pharmaceutically compatible carriers and solvents. The treatment can also be exposure to various kinds of energy or particles, such as gamma-irradiation, or can be a combination of approaches. In some cases, the treatment can also be administration of one or more substances or exposure to conditions, or a combination of both, wherein the effects of the treatment as anti-cancer therapy are unknown.

Preferably, mammals, e.g., mice, used in the methods of the invention comprise a population of cells engineered to become hematopoietic tumor cells such as lymphomas and/or leukemic cells. In a preferred embodiment, the mammal expresses an oncogene such as *myc* from a strong viral promoter and/or enhancer, the overexpression of which results in the development of hematopoietic tumorigenic cells and/or tumors. A preferred mammal is a mouse harboring the *Eµ-myc* gene, which comprises a *myc* gene operably linked to an *Eµ* IgH enhancer (*supra*; see, e.g., Adams et. al., Nature 318:533-538 (1985)). An overexpressed *myc* gene, such as the *Eµ-myc* gene, may be introduced into the cells of a mammal resulting in a transgenic mammal having the propensity to develop hematopoietic tumors (see, e.g., U.S. 6,583,333). Alternatively or in addition, an overexpressed *myc* gene, such as the *Eµ-myc* gene, may be introduced into the cells of a mammal using one of the vectors of the invention which further comprises a wildtype, variant, derivative or mutant form of a nucleic acid encoding a modulator or akt-, bcl-2, eIF4E, PTEN or eIF3E activity and which can infect or transduce a stem cell of the mammal that will develop into cells of the hematopoietic lineage. More preferably, the stem cell is a hematopoietic stem cell (pluripotent or multipotent). In another preferred embodiment, the mammal comprise tumorigenic cells comprising a vector of the invention but has non-transgenic stem cells (e.g., produced by transplanting a transgenic tumor cell of the invention into a host syngeneic or sub-lethally irradiated mammal). ,

In certain preferred embodiments, activated myc in combination with akt, bcl-2 and/or eIF4E activities are introduced, individually or in any combination, into stem cells or tumorigenic cells of the mammal by means of a viral vector. The mammal, usually a mouse, readily develops primary hematopoietic tumors of a uniform genotype, i.e., *Eµ-myc* in combination with one or more of the activated akt, bcl-2, eIF4E or repressed PTEN or eIF3E genes. Such mice are useful research tools for identifying genes, mutations, genetic alterations, treatments, agents and conditions which alter the expression of these and other genes and activities involved in cell growth, chemosensitivity through apoptotic signaling pathways, and the development of resistance to such treatments through genetic alterations that affect those signaling pathways.

In another embodiment, a test treatment may be administered *in vitro* to hematopoietic tumor cells, such as lymphoma cells, that originally arose in a mouse engineered to develop a hematopoietic tumor. The tumor cells express activated akt, bcl-2 or eIF4E activity or repressed PTEN or eIF3E activity (or any combination thereof). Treated tumor cells are monitored for growth, wherein slowing or arresting of growth indicates sensitivity of the cell to the test treatment. As before, in preferred embodiments, the hematopoietic tumor cells also express activated *myc* from a strong viral promoter/enhancer such as an *E*µ IgH enhancer.

Thus another embodiment of the invention provides a method for testing a hematopoietic tumor cell such as a lymphoma for sensitivity to a treatment, wherein the lymphoma arose in a mouse that received cells from *Eµ-myc* hematopoietic stem cells transduced with a pMSCV-Akt vector for sensitivity to a treatment. Lymphoma cells are cultured *in vitro,* a treatment is administered to the cells (e.g., a drug is contacted with the cells), and the cells can be monitored for growth (e.g., by observing cell number, confluence in flasks, staining to distinguish viable from nonviable cells). A failure to increase in viable cell number, a slower rate of increase in cell number, or a decline in viable cell number, compared to cells which have been left untreated, or which have been mock-treated, is an indication of sensitivity to the treatment.

In certain preferred embodiments, activated akt, bcl-2 and/or eIF4E and/or repressed PTEN and/or eIF3E activities are introduced, individually or in any combination, into stem cells or tumorigenic cells of the mammal by means of a viral vector, preferably before the test treatment. In certain preferred embodiments, the viral vector is a retroviral vector which is capable of infecting or transducing stem cells which produce hematopoietic cells, such as MMLV- and MSCV-derived retroviral vectors. Lentiviral vectors which can transduce stem cells that produce hematopoietic cells are also preferred. Any of the above described vectors of the invention, as are those which are used in the Examples set forth herein, may be used in this and other methods of the invention.

In other embodiments, the invention provides methods for identifying a treatment that increases sensitivity of a hematopoietic tumor cell to a chemotherapeutic drug or agent. The methods involve administering a test treatment to a non-human mammal (e.g., a mouse) that has been engineered to develop a hematopoietic tumor. The mammal also comprises a population of hematopoietic cells in which one or more of akt, bcl-2 or eIF4E activity has been activated, or PTEN or eIF3E activity has been repressed. The mammal is monitored for the development of hematopoietic tumors and the extent to which such tumors are present in the mammal after the test treatment is compared to the extent to which such cells are present in a control mammal. If tumor onset occurs less frequently in the test mouse than in the control mammal, the treatment is one which increases chemosensitivity in an akt-, bcl-2, or eIF4E-activated cell to the test treatment. Likewise, if remission from the primary hematopoietic tumors occurs more frequently in the test mammal than in the control mammal, the treatment is one which increases chemosensitivity in an akt-, bcl-2, or eIF4E-activated tumor to the test treatment. In contrast, if tumor onset occurs more frequently in the test mammal than in the control mammal, or if remission from the primary hematopoietic tumors occurs less frequently in the test mammal than in the control mammal, the treatment is one which decreases chemosensitivity in an akt-, bcl-2, or eIF4E-activated cell to the test treatment.

Thus a further embodiment of the invention provides a method employing secondary tumors arising from transplanted primary tumor cells (e.g. see Examples 2, 9-11). In this way, a statistically significant number of mice with the same lymphoma can be studied for their response to a regimen of therapy. One or more primary tumors can be harvested from an animal and transplanted into recipient mice by a suitable method, such as by tail vein injection. After a period of time, secondary lymphomas arise in the recipient mice. A treatment is then administered to one population of recipient mice which have developed tumors (making a "treated" population). A second population of recipient mice can serve as controls and remain untreated or be sham-treated. Thereafter, the treated population of mice is monitored for remission (ordinarily, by palpation for tumors). The proportion and length of remissions among the treated population indicate the extent of sensitivity of the lymphomas to the administered treatment(s). If relapse occurs, and further tumors arise, the process can be repeated. Lymphomas arising upon relapse can be collected for the study of resistance to the therapy.

The invention further provides a method for detecting a genetic alteration in a cell, the alteration which is associated with resistance to a treatment that normally causes remission of a tumor. Tumor cells are harvested from a mammal (e.g., a mouse) that is or which has previously been engineered to develop hematopoietic tumor cells (e.g., which overexpresses myc, as described above). The mammal is also engineered to have a population of hematopoietic cells (e.g., by transducing stem cells or primary tumor cells) characterized by an activated akt, bcl-2 or eIF4E activity (or any combination thereof), and/or repressed PTEN and/or eIF3E activities. Tumor cells are transplanted into recipient mice and tumors permitted to arise. The recipient mice are then treated with a therapeutic agent, thereby achieving remission, and the recipient mice are monitored for relapse following remission. Secondary tumor cells are isolated from recipient mice. In some embodiment, tumor cells are optionally passaged through different recipient mice, one or more times. A measurable difference in the level or function of a gene or gene product between the tumor cells of the last performed step and primary tumor cells of the first step is indicative of a genetic alteration in the subsequently formed tumors.

The step of identifying a difference in the level or function of a gene product may be performed by any of numerous methods well known in the art, preferably using high throughput methods and arrays or microarrays. Such methods may be designed to monitor RNA expression (e.g., by nucleic acid hybridization and other such techniques). In addition, methods which measure protein expression may also be used. As described above, in preferred embodiments, tumor cells of a well-defined genotype are used in the methods. Preferably, the tumor cells express activated myc, and one or more of the following: activated akt, bcl-2, eIF4E or repressed PTEN or eIF3E activities.

In preferred embodiments, stem cells are engineered by introduction of an activated akt-, bcl-2, and/or eIF4E (alone or in combination), and/or a repressed PTEN and/or eIF3E. One or more of these activities may be introduced into a stem cell which expresses activated myc (or another oncogenic activity) which results in formation of hematopoietic tumors in a mammal. Alternatively (but not mutually exclusive), activated myc may be introduced in conjunction with one or more of the activated akt-, bcl-2, and/or eIF4E (alone or in combination), and/or a repressed PTEN and/or eIF3E functions on a viral vector, such as a retroviral vector, as described above. Any of the vectors of the invention, and cells transduced with them, may be useful in such methods. Preferably, the stem cells are human cells, and more preferably, are hematopoietic stem cells. In certain embodiments, primary tumor cells are transplanted into new recipient mice and tumors are allowed to form. A treatment is then administered to recipient mice, thereby achieving tumor remission. Mice are monitored for the appearance of tumors (relapse) and secondary tumor cells are harvested from recipient mice. The steps of transplanting, treating to achieve remission and monitoring secondary tumor formation are optionally repeated in sequence one or more times, using different recipient mice with each repetition.

Methods for obtaining hematopoietic tumor cells for the study of drug resistance associated with a test gene (or genetic alteration) are also provided. In one such method, a treatment is administered to mice engineered to form hematopoietic tumorigenic or tumor cells (e.g., mice comprising a population of cells having a genome comprising a *myc* gene operably linked to an *Eµ* IgH enhancer); and further comprising the test gene by insertion into the genome of a retroviral vector (e.g., pMSCV) bearing the gene. Such mice develop a hematopoietic tumor or a pre-malignant condition due to the activated *myc* gene, and the treatment causes remission of the tumors or pre-malignant cells in the mice. The mice are monitored for tumor relapse and steps are optionally repeated in sequence one or more times. Hematopoietic tumor cells, such as lymphoma cells, are then harvested from the mice of the last performed step. Harvested cells are useful, e.g., in methods of the invention involving screening for genes and therapeutic agents which affect tumorigenesis and apoptosis-related pathways involved in the control of chemoresistance in tumor cells, especially hematopoietic tumor cells. One advantage of such isolated tumor cells is that they are of a relatively well-defined or known genotype, as the mammalian model is one which is engineered to develop hematopoietic tumors (e.g., by overexpression of an oncogene such as *myc*), and has an activated akt, bcl-2 and/or eIF4E activity and/or a repressed PTEN and/or eIF3E activity to create chemoresistance in the model.

In another embodiment, the invention provides methods for testing the effects of a treatment on tumor growth by transducing (*Eµ-myc*) hematopoietic stem cells with a vector comprising an activated akt, bcl-2, and/or eIF4E activity (or repressed or mutated PTEN or eIF3E activity); administering such transduced cells to irradiated recipient mammals, wherein the recipient mammals develop primary tumors; harvesting a primary tumor from an irradiated animal; administering cells derived from the primary tumor to a recipient animal; administering a treatment to the recipient animal; and monitoring the recipient animal for the effect of the treatment on tumor growth. In this embodiment, if tumor growth is decreased or increased in the recipient animals relative to tumor growth in control animals, there is an inhibitory or enhancing effect, respectively, of the treatment on tumor growth. Preferred embodiments of this method parallel the preferred embodiments of methods described above, insofar as any vector or cell of the invention may be used. In one preferred embodiment, the treatment comprise an agent that is a member of a chemical library.

The invention further provides a method for testing a nucleic acid for its effect on tumorigenesis and/or sensitivity of a tumorigenic cell to a treatment. The method involves lethally irradiating a recipient mammal; introducing a test gene into stem cells engineered to produce hematopoietic tumors in the recipient mammal; reconstituting hematopoietic cells of the recipient mammal with such altered stem cells; and observing the effect of the test gene on tumorigenesis in the recipient mammal. Preferably, the stem cells are engineered to produce hematopoietic tumors in a mammal by means of an activated myc gene. In another preferred embodiment, a primary tumor cell (rather than a stem cell) is transduced with the test gene and introduced into a syngeneic and/or sub-lethally irradiated mammal. In certain preferred embodiments, the test gene is introduced into a stem cell or tumor cell before or in combination with a nucleic acid encoding activated *myc.* Thus, the starting stem cell or tumor cell may but need not be a cell genetically engineered to overexpress myc. In certain preferred embodiments, the stem cell or tumor cell is derived from a patient and the methods of the invention used to diagnose which therapy combination will be most effective in preventing or diminishing tumor onset and/or in preventing the development of chemoresistance in an akt-, bcl-2-, eIF4E-activated hematopoietic tumor cell.

In a more preferred embodiment, a nucleic acid encoding an activated akt, bcl-2 or eIF4E activity, and/or a repressed PTEN and/or eIF3E activity, is also introduced into the stem cell or tumor cell, alone or in any combination. One of more of these activities may be introduced into a myc-expressing cell, or myc activity may be introduced in conjunction with activation of the akt-, bcl-2, eIF4E signaling pathway. Further, the test gene may be administered together or separately from the component(s) of the akt-, bcl-2, eIF4E signaling pathway. In another preferred embodiment, the test gene is introduced into stem cells or tumorigenic cells as a member of a collection of nucleic acid molecules contained in a library. The library can be any of a number of nucleic acid libraries, including cDNA or synthetic expression libraries, shRNA (RNAi) inhibitory libraries and the like.

A further embodiment of the invention is a test for the effect of a gene on sensitivity of a lymphoma to a treatment (e.g., anti-cancer drug). In one embodiment, cells can be produced from a lymphoma described herein. A gene to be tested for its effect on the sensitivity to a treatment of the lymphoma is introduced into the cells, such that the cells produce the gene product.

Introduction of the gene can be, for instance, by transformation, such as by electroporation, by calcium phosphate, DEAE-dextran, or by liposomes, using a vector which has been constructed to have an insertion of the gene. See chapter 9 in Ausubel, F.M. et al, Current Protocols in Molecular Biology, containing supplements through Supplement 63, (2003), John Wiley & Sons, New York.

The introduction of a gene of interest can also be accomplished by viral infection, for example, by a retrovirus. Retroviral gene transfer has been used successfully to introduce genes into whole cell populations, thereby eliminating problems associated with clonal variation (McCurrach, M.E. et al., Proc. Natl. Acad. Sci. USA 94:2345-2349, (1997); Samuelson, A.V. and Lowe, S.W., Proc. Natl. Acad. Sci. USA: 12094-12099, (1997); Serrano, M. et al., Cell 85:27037, (1997)).

Tumor cells so altered or transformed by the introduced gene, or unaltered cells, ("unaltered" including cells which have been transformed with a control vector, transfected with control DNA, or infected with a control virus (control constructs not carrying the gene of interest), can be introduced into immunocompetent recipient mice ("test" mice receiving the gene and "control" mice not receiving the gene). Cells can be introduced by injection, for example, by injection into the tail vein of the mice. Lymphomas are allowed to form in both the test and control mice, and both test and control mice are monitored for the development of lymphomas (for example, by palpation for tumors, or by detection of green fluorescent protein in tumors genetically altered to produce GFP). Both groups of mice are administered a treatment, preferably a drug given at a dose with a known anti-tumor effect, and monitored for remission. A difference in the frequency of remissions in the test mice compared to that of the control mice (remissions are not anticipated in control mice), indicated by an increase in the percentage of tumor cells expressing the gene in treated compared to untreated mice), indicates an effect of the gene on the response of the lymphoma to the treatment. This method can be used to look for test genes which increase or decrease the effectiveness of a test treatment on tumor formation.

This method can determine what the response of an animal would be to the same therapy, with and without the gene. Thus, genes that are important to drug sensitivity of a lymphoma, or analogously, to another tumor cell type, can be identified.

In yet another preferred embodiment, the invention provides a method for inhibiting growth of an akt-, bcl-2-, and/or eIF4E-activated tumor cell in a mammal, by administering an effective dose of rapamycin or active analog thereof in combination with a chemotherapeutic agent. The chemotherapeutic agent is preferably one (or more) selected from the group consisting of adriamycin, cyclophosphamide and doxorubicin.

In another preferred embodiment, the invention provides a method for assessing the sensitivity of a tumor to a test chemotherapeutic agent. The method involves determining whether the tumor expresses activated akt, bcl-2 or eIF4E activity; and treating such an activated tumor with rapamycin or an active analog thereof in combination with the test chemotherapeutic agent. The tumor is sensitive to the test chemotherapeutic agent if regression or remission occurs following the treatment in comparison to control treatments. Preferably, the test chemotherapeutic agent is a DNA modifying agent such as one of a number of DNA damaging agents, DNA cross-linking agents, DNA alkylating agents and topoisomerase inhibitors that cause DNA damage. In more preferred embodiments, the agent is cyclophosphamide, melphalan, doxorubicin or daunorubicin. In other preferred embodiments, the test chemotherapeutic agent is expressed as a member of a nucleic acid or a chemical library.

In yet another aspect, the invention provides a method for inhibiting the growth of akt, bcl-2 or eIF4E-activated tumors in a mammal, comprising administering to the mammal an effective dose of an inhibitor of translation in combination with an effective dose of one or more chemotherapeutic agents. Preferred inhibitors of translation include but are not limited to: 4E-BP(1, 2 or 3); tsc-1 and tsc-2; the N-terminal domain or a dominant-negative mutant of eIF4G (Morino et al., Mol Cell Biol. 20(2):468-77 (2000)); a dominant-negative mutant of eIF4A (Oguro et al., RNA 9(4):394-407 (2003); Pdcd4 (Yang et al., Mol Cell Biol. 23(1):26-37 (2003); picornavirus protease 2A (Haghighat et al., J. Virol. 70(12):8444-50 (1996); and NSP3 (Padilla-Noriega et al., Virology 298(1):1-7 (2002)).

In other aspects, the invention provides a method for identifying a potential target for drug therapy in the treatment of an akt, bcl-2 or eIF4E-activated tumor, the method comprising harvesting hematopoietic tumor cells from a mouse engineered to develop a hematopoietic tumor and bearing a population of cells comprising an activated akt, bcl-2 or eIF4E activity (or repressed PTEN or eIF3E activity); and introducing a nucleic acid library into a population of the harvested cells. A fraction of cells comprising one or more members of the nucleic acid library is transplanted into recipient mice; and hematopoietic tumor cells allowed to arise in transplanted recipients. Earlier recurrence and reduced survival of a recipient mouse compared to control mice indicates the presence of a potential target encoded by at least one member of the introduced nucleic acid library. The potential target may be characterized further by well known methods, including DNA sequencing, genetic analyses, expression array technology (at the nucleic acid and/or protein level); two-hybrid screening and the like.

The invention further provides a method for identifying a drug for treatment of an akt, bcl-2 or eIF4E-activated tumor, comprising harvesting hematopoietic tumor cells from a mouse engineered to develop a hematopoietic tumor and bearing a population of cells comprising an activated art, bcl-2 or eIF4E activity (or repressed PTEN or eIF3E activity); making extracts of such hematopoietic tumor cells and treating them with one or more test molecules. The effects of the test treatment on one or more markers of the akt-, bcl-2, eIF4E signaling pathway are assessed by conventional means. A change in the phenotype of a marker of the akt signaling pathway in the direction of decreased akt, bcl-2 and/or eIF4E activity indicates the presence of a potential drug. Likewise, a change in the phenotype of a marker of the akt signaling pathway in the direction of increased PTEN activity indicates the presence of a potential drug. Markers of the akt signaling pathway include akt, p-akt, TSC1/2, p-TSC1/2, Rheb, p-Rheb, mTOR, p-mTOR, s6 kinase, p-s6 kinase, S6, p-S6, 4E-BP and p-4E-BP, eIF4G, p-eIF4G, eIF4B, p-eIF4B, bcl-2, p-bcl-2, FKHR, p-FKHR, bad, p-bad, GSK, p-GSK, eIF2B, p-eIF2B. Markers of the akt signaling pathway also include caspase-9 phosphorylation, bad cytoplasmic localization, mdm-2 phosphorylation and cytoplasmic localization, p27 cytoplasmic localization, forkhead transcription factors cytoplasmic localization, changes in glucose metabolism and reduced apoptosis.

Conventional methods may be used to assess the presence of such markers in test- and control-treated animals or cell extracts. Such methods include but are not limited to immunoblotting, immunofluorescence, immunohistochemical staining, co-immunoprecipitation with cytoplasmic proteins (e.g. 14-3-3 proteins); functional assays for metabolic markers (e.g., high glucose uptake by PET, etc.), apoptosis, reduced p53 induction following DNA damage/stress, measures of RNA levels such as Northern blot analysis, RT-PCR (e.g., quantitative, Real-Time [RT] PCR).

The invention described herein exploits mouse models to generate tumors with specific genetic alterations, allowing the production of useful tools for future drug discovery programs applicable to human lymphomas. By understanding the regulation and execution of apoptosis in tumor cells, it may be possible to selectively increase the chemosensitivity of tumor cells, or to develop novel therapies to activate apoptosis more directly. The results of terminal deoxynucleotidyl transferase-mediated dioxygenin-11-dUTP nick end labeling (TUNEL) and Ki67 analyses of histological sections from *Eµ-myc*/Akt and *Eµ-myc*/Bcl-2 tumors (see Examples herein) suggest that Akt, like Bcl-2, interferes with apoptosis.

The approach of introducing the Akt oncogene into hematopoietic stem cells by retroviral vector creates a valid and relevant model for the *in vivo* evaluation of agents targeting the PI3K/Akt pathway. Specifically, it can be useful in target validation, selection of potent inhibitors in a physiological setting, and the design of therapeutic combination regimens most active in cancers that display high levels of Akt activity. For example, the approach can be used to test whether rapamycin - an inhibitor of the Akt target mTOR - will be effective against the Akt-overexpressing lymphomas, or reverse their profound resistance to conventional agents. The approach is more broadly applicable to other genes, for example, genes suspected of encoding gene products with a role in tumor maintenance, or a role in apoptosis as effectors, inhibitors, or the like.

Lymphoma cells - primary or secondary - generated by the methods described herein can be studied for differences in the level of gene copy number or gene expression by a number of methods known in the art. For example analysis of polymerase chain reaction (PCR) products using lymphoma cell DNA as template, and primers to amplify genes of interest. PCR can be combined with capillary electrophoresis (CE), as in integrated PCR-CE on a microchip. Recent technology enables one to detect variation in genome copy number by identifying small deletions or amplifications of genes under various treatment conditions (see Lucito R., et al., "Representational oligonucleotide microarray analysis: a high-resolution method to detect genome copy number variation", Genome Res., 13(10):2291-305 (2003)). One may also use genomic arrays such as CGH or ROMA to analyze gene copy number. DNA microarrays can be used to identify gene expression patterns and to detect differences in such patterns, for example, with the aid of software designed for the purposes of such comparisons. Differences in gene function can be analyzed by RNA interference, for example, as in Mousses S. et al., Genome Res. 13(10):2341-2347 (2003). Other methods to study differences in gene expression resulting from mutations in the lymphoma cells are those that analyze the proteins produced by the cells. Proteins produced in cells can be separated and identified by protein profiling, e.g., by 2-D gei electrophoresis and mass spectrometry. Protein arrays can be produced, and proteins can be characterized and identified, using, for example, matrices of peptides, antibodies, apatamers, or chromatographic surfaces. Profiles of the DNA, RNA or proteins produced by such methods can be compared between primary lymphomas sensitive to a treatment and secondary lymphomas that have acquired one or more mutations to become resistant to the treatment.

Our results provide new insights into Akt signaling and tumor behavior. We find that Akt lymphomas are phenotypically similar to those harboring a purely anti-apoptotic oncogene with respect to their accelerated onset, aggressive pathology, ability to retain p53 and chemotherapy resistance. Thus, despite its pleiotropic activities, the pro-survival functions of Akt appear sufficient to promote tumorigenesis. We further show that the mTOR inhibitor rapamycin can restore apoptotic sensitivity to lymphomas expressing Akt, and that the translation factor eIF4E can recapitulate Akt action *in vivo.* Consequently, a substantial proportion of Akt survival signaling may result from deregulated translation, perhaps through altering the recruitment of pro- and anti-apoptotic mRNAs to polysomes (Grolleau, A., et al., "Global and specific translational control by rapamycin in T cells uncovered by microarrays and proteomics," J. Biol. Chem. 277: 22175-22184 (2002) and Rajasekhar, V. K., et al., "Oncogenic Ras and Akt signaling contribute to glioblastoma formation by differential recruitment of existing mRNAs to polysomes," Mol. Cell. 12: 889-901 (2003)). Of note, the apparent role of translational control in cell survival extends to tumors with other PI3K pathway lesions, since the chemotherapy resistance of PTEN-deficient lymphomas is reversed by rapamycin in a manner that is blocked by eIF4E. Together, these results provide *in vivo* evidence that translational signaling through Akt is a broadly relevant oncogenesis and drug resistance mechanism. They also provide the first evidence that overexpression of eIF4E promotes oncogenesis and drug resistance.

The invention further provides a means for directly comparing eIF4E to Akt in the *Eµ-myc* model, in order to further examine the effects of translational control on tumor phenotypes. In one preferred embodiment, the encoded eIF4E polypeptide has altered biological activity. In certain embodiments, it is preferred that eIF4E have enhanced or overexpressed activity. Example 11, for example, shows how hematopoietic reconstitution with *Eµ-myc* transgenic HSCs expressing eIF4E accelerate lymphomagenesis in a manner highly similar to Akt overexpression. Moreover, the invention further provides a method for testing the chemosensitivity of eIF4E tumor cells by quantifying the disseminated pathology and proliferation/apoptosis ratio of the lymphomas.

In another embodiment, the slowing or arresting of tumor growth in mice having one or more hematopoietic tumors characterized by cells having altered (i.e. increased) eIF4E activity can be tested for by administering a drug treatment. The said treatment comprises a chemotherapeutic agent which causes remission of hematopoietic tumor cells. The time to tumor relapse or remission can be used as a measure of chemoresistance or chemosensitivity. Example 11 provides data showing that the eIF4E transgene confers a high degree of resistance to various chemotherapeutic treatments, including rapamycin and doxorubicin.

The invention further describes a mouse model to generate tumors with specific genetic alterations, wherein the mice show a mutated (i.e. altered; increased or decreased) expression of PTEN or eIF4E relative to wild-type control mice. In a preferred embodiment, *Eµ-myc*/*PTEN*+/- mice are derived from a cross of a transgenic *Eµ-myc* mouse to a transgenic PTEN+/- mouse and the method includes detection of a loss of heterozygosity in the PTEN locus by allele specific PCR. Example 12 illustrates an enhanced susceptibility to tumors in the PTEN heterozygous mice, and further characterizes an enhanced chemoresistance in these mice in vivo. PTEN heterozygosity thus causes a significant acceleration and increased penetration in lymphoma development in vivo. The invention further describes a lymphoma development in *Eµ-myc*/*PTEN*+/- mice that is resistant to chemotherapy, namely doxorubicin, and reversed by rapamycin.

A further embodiment of the invention is a method for transducing HSCs into recipient *Eµ-myc*/*PTEN*+/- mice wherein the HSCs are infected with a retroviral pMSCV-eIF4E vector, or with the particular pMSCV-eIF4E vector, pMSCV-eIF4E-IRES-GFP. A transduction procedure is outlined in Example 13. A preferred embodiment characterizes a method for quantifying changes in chemoresistance in eIF4E infected tumor cells derived from said *Eµ-myc*/*PTEN*+/-mice, wherein eIF4E confers resistance to *Eµ-myc*/*PTEN*+/- tumors after in vivo drug treatment (see Example 13).

The invention further provides a method for harvesting *Eµ-myc* HSCs and infecting them in vitro with a pMSCV-eIF4E vector, or with the particular pMSCV-eIF4E-IRES-GFP vector expressing different mutants of eIF4E. A preferred embodiment includes mutants of various residues of proteins that are important in the Akt pathway and more preferably that are important in the function of eIF4E. Example 14 shows that mutating some of these proteins, namely E103A, S209A, and W56A, can affect eIF4E's oncogenicity in vitro. In particular, S209 induces tumors at lower and much delayed penetrance. This would indicate that the phosporylation site S209 is important for eIF4E's oncogenicity. Interestingly, the S209A mutant affects eIF4E function without affecting its interaction with 4E-BP. The invention thus provides a method of mapping functional domains of a protein that play a role in oncogenesis and/or chemoresistance. As phosphorylation at this site is mediated via p38 and MEKK activation of MNK-1, this invention also indicates, that eIF4E tumors (and similarly Akt expressing tumors) should be sensitive to pharmacological inhibitors of these kinases.

The invention further provides a method to investigate whether RNAi applied to a well-characterized gene can stably suppress gene expression in HSCs and produce detectable phenotypes in mice. A retroviral mediated gene transfer is illustrated in Example 15. Herein *Eµ-myc* mice are infected with a short hairpin against PTEN (shPTEN). Infection occurs by transduction of cells with a pMSCV-shPTEN vector. A preferred embodiment is the use of a particular pMSCV-shPTEN-IRES-GFP vector. Two murine PTEN sequences can be used, as described and disclosed in Example 15; PTEN sh1 and PTEN sh2. Transduced cells integrate the pMSCV-shPTEN-IRES-GFP vector in the cell genome and induce lymphomas in the *Eµ-myc* mouse model.

The invention also encompasses a method for testing the chemoresistance of cells that have been transduced with pMSCV-shPTEN vectors. The time to tumor development is shortened following reconstitution of lethally irradiated mice infected with the pMSCV-shPTEN vectors. Moreover, PTEN expression is downregulated in the presence of shPTEN, as illustrated by a decreased expression of endogenous total and phosphorylated PTEN by western blotting in Example 15. The combination of vector, cells and methods that allow the introduction of shPTEN RNAi into the system to knockdown PTEN and create activation of endogenous Akt is a further embodiment of the invention that enables a broader analysis of loss of function changes in the *Eµ-myc* mouse model.

Accordingly, the present invention is also directed to modulating the activity of translation factors in the treatment of diseases, conditions and states associated with abnormal proliferative growth phenotypes, especially those which are lymphoproliferative or myeloproliferative. The invention provides compositions and methods for preventing Akt, Bcl-2 or eIF4E-mediated inhibition of apoptosis to increase chemosensitivity of tumor cells such as those expressing activated Akt signaling components.

In one embodiment, the invention thus provides inhibitors of eIF4E activity in a cell. Also provided are cells in which eIF4E activity is inhibited, and mammals comprising such cells, particularly hematopoietic cells and more particularly, hematopoietic stem cells. The present invention also provides screening methods for identifying agents that will inhibit eIF4E activity, thereby deregulating the akt pathway signaling which affects apoptosis and chemosensitivity or resistance. A preferred inhibitor of eIF4E activity is 4E-BP (binding protein), which is a repressor of eIF4E activity. 4E-BP inhibits eIF4E activity by sequestering eIF4E in an inactive form. eIF4E thus shuttles between an unbound state or a state where it is bound to eIF4G (and participates in translational initiation), and the 4E-BP bound, inactive state. Accordingly, overexpression of 4E-BP in a cell of the invention will modulate apoptosis and decrease akt-/bcl-2-/eIF4E-mediated chemoresistance in a tumor cell *in vivo.*

It is also envisioned that inhibiting other general translation factors (i.e., molecules which participate directly in initiating translation of mRNA into protein) tumor cells such as hematopoietic tumor cells will have the desired effect of increasing apoptosis and reducing chemoresistance. Examples of suitable targets for inhibition include the translation factors eIF3 (subunits thereof), eIF1, eIF5, eIF2 (subunits thereof), eIF2B, eIF1A, eIF4A (isoforms I, II and III), eIF4B, eIF4G (isoforms I and II), eIF4H, eIF4F (which is a complex of eIF4E, 4A, and 4G), eIF5A, eIF5B, and elongation factors eEF1A1, eEF1A2, eEF2, as well as the eIF4E related protein, eIF4E-HP, and the eIF4E transporter protein, eIF4E-T. Also included are the poly A Binding Protein (PABP) and translational termiantion factors such as eRF1 and eRF3 (eukaryotic release factors 1 and 3). See, e.g., Table 1.

**Table 1**

| Eukaryotic protein synthesis inhibitors | | | |
|---|---|---|---|
| **Inhibitor** | **Site of Inhibition^{a}** | **In Vivo Inhibition^{b}** | **In Vitro Inhibition** |
| Arginine-Aminoglycoside Conjugates | Elongation | - | + |
| 4-Aminohexose Pyrimidine Nucleosides | Transpeptidation, Agonist of Stress-Activated Protein Kinase | + | + |
| Anisomycin | Transpeptidation, agonist of Stress-Activated Protein Kinase | + | + |
| Aurintricarboxylic acid | Ternary Complex Formation | - | + |
| Baciphelacin | Not Known | + | + |
| Bouvardin | EF-2 dependent translocation | + | + |
| Clotrimazole | eIF-2a phosphorlylation, Depletion of Ca++ stores | + | n.t. |
| Cryptopleurine | Transpeptidation | + | + |
| Didemnin | eEF-1α | + | + |
| Diterpene Esters (Genkwadaphnin, yuanhuacine) | Peptidyl transferase | + | + |
| Edeine | Initiation and elongation by interfering with P site function | ?? | + |
| Eicosapentaenoic Acid | Phosphorylation of eIF2a | + | - |
| Emetine Family | Translocation | + | + |
| Flavinoids | Phosphorylation of eIF2a | + | n.t. |
| Fusidic acid | Translocation | - | + |
| Glutarimide Family (e.g. -cycloheximide) | Translocation | + | + |
| Harringtonine Family | Met-tRNAi binding | + | + |
| 2-(4-methyl-2,6-dinitro-anilino)-N-methyl-propionamide (MDMP) | Initiation | + | + |
| M'GDP | Initiation | - | + |
| Nagilactone | Elongation | + | + |
| Negamycin | Initiation, miscoding, termination | + | + |

| | | | |
|---|---|---|---|
| ^{a}Refers to the specific partial reaction inhibited or otherwise affected. ^{b}n.t.-not reported or not known. | | | |

Our results also have profound implications for considering the use of targeted therapeutics alone or in combination with conventional chemotherapeutic agents. The result that rapamycin can reverse drug resistance in Akt-expressing tumors is compelling evidence that reversing apoptotic defects can restore drug sensitivity *in vivo.* Furthermore, the ability of eIF4E to mediate drug resistance downstream of Akt implies that inhibitors of translation initiation may also be effective chemosensitizing agents, and perhaps less prone to resistance. The selectivity of rapamycin to reverse drug resistance in Akt expressing lymphomas - despite their broad similarities to those with other apoptotic lesions - implies that the utility of this combination treatment would be missed in clinical trials based solely on tumor type or pathology. Together, these results provide *in vivo* validation for a strategy to reverse drug resistance in human cancers, and underscore the importance of tailoring cancer therapy based on tumor genotype.

The invention further provides methods for treating a disease, condition or disorder associated with abnormal regulation of cell proliferation, especially abnormal lymphoproliferative or myeloproliferative states. Diseases and disorders include but are not limited to: Non-Hodgkin's lymphoma (NHL) and Hodgkin's lymphoma; follicular lymphoma (FL); small cell lymphoma, diffuse large cell lymphoma; Burkitt's lymphoma; B cell chronic lymphocytic leukemia (B-CLL); T cell lymphocytic leukemia (T-CLL); B cell acute lymphocytic leukemia (B-ALL); T cell acute lymphocytic leukemia (T-ALL); chronic myelogenous leukemia (CML); myelodysplastic syndrome (MDS); promyelocytic leukemia (PML); acute myelogenous leukemia (AML); autoimmune-lymphoproliferative syndrome (ALPS); multiple myeloma (MM); and plasmacytoma.

The following are examples which illustrate the compositions and methods of this invention. These examples should not be construed as limiting: the examples are included for the purposes of illustration only.

### EXAMPLES

### Methods:

### Isolation of tumor cells and genotyping PCR

Lymphoma-bearing *E-myc* transgenic mice (C57BL/6 inbred strain) were sacrificed by CO₂ euthanasia. Dissected lymph nodes were minced in phosphate buffered saline (PBS) and filtered the material through a 35-m nylon mesh. The tumor cells were sorted for green fluorescent protein (GFP) content by flow cytometry (FACScalibur, Beckton Dickinson). DNA was isolated from the purified GFP positive population by standard procedure and loss of the remaining wild-type allele [loss of heterozygosity (LOH)] of p53 or PTEN in tumors originating from p53 or PTEN heterozygous hematopoietic stem cells (HSC), respectively, was detected by allele-specific PCR as described (Schmitt, C.A. et al., Genes Dev 13(20):2670-2677, (1999); Di Cristofano A., et al., Nature Genetics, 19(4):348-355, (1998)).

### In Vivo Chemotherapy

Tumor cells were transplanted from the Akt-expressing tumors and from control tumors (*Eµ-myc, arf*^{-/-},*p53*^{+/+}) into syngeneic, non-transgenic 6-10 week old C57BL/6 mice by tail vein injection [10⁶ viable lymphoma cells in phosphate-buffered saline (PBS)] and recipient mice were monitored for tumor formation twice a week by palpation of the pre-scapular and cervical lymph-nodes. When tumors became well palpable, adriamycin (ADR) or cyclophosphamide (CTX) was given at a single dose of 10 mg/kg or 300 mg/kg body weight intraperitoneally (i.p.), respectively. Following treatments, mice were monitored for immediate response and time to relapse by lymph node palpation and by blood smears (by tail artery bleeding) stained according to a modified Wright's protocol using the Leukostat kit (Fisher Scientific, Pittsburgh, Pennsylvania). The data were statistically evaluated.

A 'complete remission' (CR) was defined as absence of any palpable tumor and the absence of leukemia, as examined by blood smears. 'Tumor free survival' was defined as the time between treatment and reappearance of a well-palpable lymphoma (Schmitt, C. A., et al., "Genetic analysis of chemoresistance in primary murine lymphomas," Nat. Med. 6: 1029-1035 (2000)). 'Overall survival' was defined as the time between treatment and progression to a terminal stage at which the animals were sacrificed. Tumor free and overall survival data were analyzed in the Kaplan-Meier format using the log-rank (Mantel-Cox) test for statistical significance. Whole body fluorescence imaging of living animals was performed as described (Schmitt, C. A., et al., "Dissecting p53 tumor suppressor functions in vivo," Cancer Cell 1: 289-298 (2002) and Yang, M., et al., "Whole-body optical imaging of green fluorescent protein-expressing tumors and metastases." Proc. Natl. Acad. Sci. USA 97: 1206-1211 (2000)).

Rapamycin (RAP) was given at 4 mg/kg, i.p. x 5 d, doxorubicin (DXR) at 10 mg/kg, i.p., cyclophosphamide (CTX at 300 mg/kg, i.p., or various combinations of drugs were given. In combination studies, the cytotoxic agent was given on day 2 of the rapamycin protocol. Rapamycin (LC labs, MA) was initially dissolved at 10 mg/ml in 100% ethanol, stored at -20°C, and further diluted in an aqueous solution of 5.2% TWEEN 80 and 5.2% PEG 400 (final ethanol concentration, 2%) immediately prior to use. Doxorubicin (Sigma) and cyclophosphamide (Sigma) were dissolved in saline. In treatment studies, control lymphomas were ARF-null tumors arising in an *Eµ-myc*/*ARF-*/*-* background (Schmitt, C. A., et al., "INK4a/ARF mutations accelerate lymphomagenesis and promote chemoresistance by disabling p53," Genes Dev. 13: 2670-2677 (1999)).

### Histopathology

Tissue samples were fixed in 10% buffered formalin and embedded in paraffin. Thin sections (5 µm) were stained with hematoxylin and eosin according to standard protocols. Detection ofphosphorylated Akt [rabbit antibody against Hematopoietic stem cell-Akt Ser 473, 1:100 (Cell Signaling Technology)] and Ki-67 [rabbit antibody, 1:100 (NovoCastra)] was by standard avidin-biotin immunoperoxidase method, using biotinylated goat or rabbit specific immunoglobulins (Vector labs) at 1:500 and avidin-biotin peroxydase complexes (1:25, Vector Labs). Diaminobenzidine was used as the chromogen and hematoxylin as counterstain. For B220 immunohistochemistry [rat antibody against mouse CD45R/B220-clone RA3-6B2 (BD Biosciences, Pharmingen)], antigen retrieval was required and a biotinylated antibody against rat was used as a secondary antibody. The apoptotic rate was analyzed by TUNEL assay according to published protocols (Di Cristofano, et al., "Pten and hematopoietic stem cell27KIP1 cooperate in prostate cancer tumor suppression in the mouse," Nat. Genet. 27: 222-224 (2001)).

### Western blotting analysis

Whole-cell lymphoma cell lysates were generated by lysing and extracting cells in SDS sample buffer (60 mM Tris-HCl at pH 6.8, 10% glycerol, 2% SDS, and 5% 2-mercaptoethanol). Samples corresponding to 50 µg of protein (Bio-Rad Bradford protein assay) were separated on an SDS-polyacrylamide gel and transferred to Immobilon-Hematopoietic stem cell membranes (Millipore). Total and phosphorylated Akt and total and phosphorylated S6 kinase were detected using antibodies 9272, 9275, 9202 and 9206 respectively, (all from Cell Signaling Technology, 1:1000 dilution). Antibodies against ribosomal S6 protein (2212, 1:1000, Cell Signaling), phospho-ribosomal S6 (2215, 1:1000 Cell Signaling), cleaved nuclear poly ADP-ribose polymerase (PARP) (9548, 1:1000 Cell Signaling), Bcl-2 (N19, 1:200, Santa Cruz), eIF4E (9742, 1:2500, Cell Signaling), 4E-BP1 (9452, 1:1000, Cell Signaling), and β-actin (1:5000, Sigma) were also used as probes in western blot experiments. α-Tubulin, as loading control, was detected using the monoclonal antibody B-5-1-2 (1:5000, Sigma). Proteins were visualized using enhanced chemiluminescence (ECL, Amersham, and Lumilight, Roche) or Supersignal (Pierce). Quantification of eIF4E expression was quantified by immunoblotting using the above eIF4E primary antibody and [I¹²⁵]-recombinant protein A (at ∼0.1 µCi/ml; specific activity of 70-100 µCi/µg; PerkinElmer, Ontario, Canada) as a secondary reagent. The blot was exposed to a phosphor imager plate and scanned on a Fuji film BAS 1800II machine.

### Lymphoma cell culture and in vitro treatment

Single cell suspensions of freshly extracted lymphoma cells were plated on an irradiated (20 Gy) feeder layer (300x10⁵ NIH-3T3 cells/10-cm plate) in 45% Iscove's modified Eagle medium, 45% Dulbecco's minimal essential medium, 10% fetal bovine serum, 100 U/ml penicillin and streptomycin, 4 mM L-glutamine, and 25 µM 2-mercaptoethanol.

For western blot and immunofluorescence analysis, cells were treated with rapamycin 10 nM (Sigma) for 1 hour, then washed in PBS. Cytospin was performed using 1x10⁵ cells at 500 rpm. Cells were then fixed in 4% paraformaldhyde and analyzed for phosphorylated S6 kinase using antibody 9206 (Cell Signaling Technology, 1:200 dilution). For fluorescence detection, an Axioscop 50 immunofluorescence microscope (Zeiss, Thornwood, N.Y.) was used.

### Example 1: Generation of Eµ-myc lymphoma with defined genetic alterations

Lymphoma cells with defined genetic alterations were generated according to published procedures (see, e.g., Schmitt, C.A., et al., Cancer Cell, 1(3):289-298, (2002)). Briefly, day 13.5-18.5 pregnant mice from an *Eµ-myc-*transgenic to wildtype (C57BL/6) cross were sacrificed to obtain fetal livers, which were minced and grown at approximately 3x10⁶ cells/ml in conditions supporting hematopoietic stem cell (HSC) growth (37% DMEM, 37% Iscove's modified Dulbecco's Medium [Gibco], supplemented with 20% fetal calf serum, 2% L-glutamine [200 mM], 100 U/ml penicillin/streptomycin, 5x10⁻⁵ M 2-mercaptoethanol, 4% 0.45 µm filtered WEHI-3B supernatant, 0.2 ng/ml recombinant murine interleukin-3, 2 ng/ml recombinant murine interleukin-6, and 20 ng/ml recombinant murine stem cell factor [all cytokines from Research Diagnostics] at 37°C in a humidified 5% CO₂ atmosphere).

*Eµ-myclp53*+/- HSCs were derived from crosses of *Eµ-myc and p53*+/- mice and detection of loss of heterozygosity (LOH) in the *p53* locus was by allele specific PCR (Schmitt, C. A., et al., "Dissecting p53 tumor suppressor functions in vivo," Cancer Cell 1: 289-298 (2002) and Schmitt, C. A., et al., "INK4a/ARF mutations accelerate lymphomagenesis and promote chemoresistance by disabling p53," Genes Dev. 13: 2670-2677 (1999)).

Production of retroviral supernatants and transductions were carried out as previously described (Schmitt, C.A. and S.W. Lowe, Blood Cells Mol Dis 27(1):206-216, (2001)). High-titer retroviral supernatant was passed through a 0.45 µm filter and supplemented with 4 µg/ml polybrene (Sigma). About 6x10⁶ cells were infected four times by spinoculation at 600 g for 10 min in 3 ml of retroviral supernatant every 6-8 hours. Twenty-four hours after the last infection, the fraction of GFP expressing cells was measured by flow cytometry (FACScalibur, Becton Dickinson), and typically was between 5% and 20%. Protein expression of the Akt construct was detected by western blot analysis using antibodies against Phospho-Thr308 Akt (9275, Cell Signaling Technology, 1:1000 dilution) and antibodies against HA (MMS-101P, Covance, 1:1000 dilution). α-Tubulin (B-5-1-2, Sigma, 1:5000 dilution) served as a loading control. Cells were used for reconstitution of recipient mice 2 days following the last infection.

For bone marrow reconstitution experiments 6- to 8-week old C57BL/6 recipient mice received a single 10 Gy-dose of total-body irradiation (¹³⁷Cesium source; 0.8 Gy/min), and were reconstituted 6 hours later with approximately 3x10⁶ viable fetal liver cells by tail vein injection. Mice were housed on autoclaved bedding in air-filtered cages and received neomycin-containing drinking water. Nonreconstituted, lethally irradiated mice were included as controls in every experiment, and typically had to be sacrificed between day 12 and 18 post irradiation due to bone marrow aplasia.

Upon the appearance of well-palpable lymphomas, tumors were harvested and either fixed for histological evaluation, rendered single cell suspensions and frozen in 10% DMSO or transplanted directly into normal mice for treatment studies (Schmitt, C. A., et al., "Genetic analysis of chemoresistance in primary murine lymphomas," Nat. Med. 6: 1029-1035 (2000)).

The retroviral vectors used were MSCV-GFP (the empty vector control), MSCV-Bcl-2, MSCV-Akt (myrAkt), MSCV-Akt (K179A), and MSCV-eIF4E. For in vivo competition experiments, MSCV-eIF4E was transduced into a small percentage of Akt lymphoma cells during a brief in vitro passage, and the mixed population was re-injected into several recipient mice.

pMSCV-IRES-GFP was constructed by placing GFP downstream of the pCITE1 internal ribosome entry site from encephalomycocarditis virus (IRES; Novagen) and cloning it into the EcoRI and SalI sites of the murine stem cell virus pMSCVneo (Clontech), replacing PGK and the neomycin resistance gene expressed in pMSCVneo (Van Parijs, L. et al., Immunity 11:281-288, (1999)). A gene encoding an HA-tagged, myristoylated Akt (a gift from B.A. Hemmings; Andjelkovic, M., et al., J. Biol. Chem. 272(50):31515-31524, (1997); Andjelkovic, M. et al., Proc. Natl. Acad. Sci. USA 93(12):5699-5704, (1996)) and a gene encoding a kinase-dead mutant Akt (obtained by mutating residue K179 to A179) were subcloned into pMSCV-IRES-GFP at the EcoRI and BgIII sites. Thus, pMSCV-Akt-IRES-GFP includes the 5' and 3' long terminal repeats (LTRs) from pMSCV, the ψ⁺ extended packaging signal, the human Akt gene, IRES, GFP, an origin of replication from pUC, and a gene encoding beta-lactamase, providing for ampicillin resistance. (See Figure 4).

pMSCV-Bcl-2-IRES-GFP (MSCV-Bcl-2) has been described [Schmitt, C.A. and S.W. Lowe, Blood Cells Mol Dis 27(1):206-216, 2001]. To make pMSCV-Bcl-2-IRES-GFP, Bcl-2 was cloned into the EcoRI and BgIII sites.

pMSCV-eIF4E-IRES-GFP was constructed as described above for pMSCV-IRES-GFP. A gene encoding eIF4E (GenBank Accession # XM_371067) was subcloned from pGEX-6p1-meIF4E-wt (where m is murine), and cut with XhoI, klenowed the end, ligated EcoRI linkers (NEB) to the end, re-ligated and subsequently cut out the -680 bp (all coding region) fragment. This was ligated and subcloned into pMSCV-IRES-GFP at the EcoRI site of the MSC.

Other vectors, such as pMSCV-PTEN-IRES-GFP, could be similarly constructed using the above protocol to subclone PTEN into the pMSCV-IRES-GFP vector with the 5' and 3' LTRs from pMSCV by inserting flanking sites at either end of PTEN and ligating it into a site of MSC such as EcoRI or BgIII. This method could also be used to subclone other genes into similar vectors to induce altered gene expression in vivo, by inserting the appropriate flanking sites to cut out and re-ligate into the pMSCV-IRES-GFP vector as needed.

The animals were monitored 2-3 times weekly for the occurrence of well-palpable peripheral lymph node enlargements. The time to onset data were calculated from the time of reconstitution to lymphoma onset. Statistical evaluation of tumor onset data was by log-rank (Mantel-Cox) test for comparison of the Kaplan-Meier event-time format.

Figure 2A shows the tumor onset in mice reconstituted with *Eµ-myc* HSCs transduced with control pMSCV (n=40), Akt (n=18), or Bcl-2 (n=18). An accelerated tumor onset is shown in both Akt-expressing and Bcl-2-expressing mice. Morphologically, the lymphomas resemble the *Eµ-myc* lymphomas, in that they are highly aggressive, predominantly large cell lymphomas, with a tendency for early dissemination, infiltration of non-lymphoid organs, and the frequent occurrence of an accompanying leukemia. A kinase-dead mutant Akt did not accelerate lymphomagenesis, and Akt did not cause tumor formation in non-transgenic HSCs (data not shown).

### Example 2: Tumor free survival of Akt-expressing mice in response of secondary tumors to combination therapy

Parent vector pMSCV-IRES-GFP, or derivative vectors pMSCV-Akt-IRES-GFP or pMSCV-Bcl-2-IRES-GFP were used to transduce hematopoietic stem cells obtained as described above. Irradiated recipient mice were administered the transduced hematopoietic stem cells as described above. The scheme is presented in Figure 1A.

Stem cells were infected using retroviral vectors (either pMSCV-IRES-GFP as control, pMSCV-Akt-IRES-GFP or pMSCV-Bcl-2-IRES-GFP). When primary tumors arose in these animals, the mice were sacrificed and the tumors were harvested and injected into five recipient mice. Following injection of 10⁶ cells into the tail vein, upon development of palpable (secondary) tumors (ca. 3 weeks following the iv injection), mice were treated with either adriamycin (10 mg/kg in H₂O, once, by i.p. injection) or rapamycin (4 mg/kg by i.p. injection) and adriamycin (d1 rapamycin, d2 both drugs by separate injection, d3-5 rapamycin). Mice were then palpated twice weekly for disappearance or recurrence of tumors. Data are presented as Kaplan-Meier plots in Figures 5A-5D and show the tumor free survival/time to relapse.
- Figure 5A:: Pooled data from the first 8 mice in each treatment group, receiving adriamycin treatment as described above. The tumors are from more than 5 separate primary tumors.
- Figure 5B:: Data from mice were stratified according to the genotypes.
- Figure 5C:: Treatment of Bcl-2 tumors with either adriamycin, or adriamycin and rapamycin compared to the control tumors.
- Figure 5D:: Treatment of Akt expressing tumors with adriamycin, or combination of adriamycin and rapamycin.

Data presented in Figure 6 are from the same mice treated as described above, but also include experiments using rapamycin as a single agent (same dose as above i.p. administration on days 1-5). Data are presented as percentage of treated animals that achieved a complete remission.

### Example 3: Variations in response to treatment on identical tumors

Primary tumors were produced as described above. Identical primary tumors were injected into 5 recipient animals, thus forming cohorts of mice carrying identical tumors. The treatment regimens of 7 primary Akt tumors (#135, #136 etc.) injected into multiple recipient mice were analyzed as a matched group in which identical secondary tumors received different treatments. The treatments were: adriamycin, rapamycin and the combination rapa-adr (schedule as above in Example 2); the treatment shown in Figure 7 as adr-rapa is adriamycin and rapamycin given on day 1, days 2-5 rapamycin alone at the previous doses by i.p. injection. Mice were monitored twice weekly by palpation and blood smears. The graph indicates the individual tumor free survival times, as time to relapse measured in days. If the mice were never tumor free, time to relapse is scored as '0' days.

### Example 4: Green fluorescent protein imaging of tumors in mice

Paired *Eµ-myc*/*Akt* secondary tumors were produced by injection of an Akt-GFP expressing primary tumor into 3 recipient animals. The animals were treated as described with either adriamyin d1, rapamycin d1-5 or rapa d1, adr+rapa d2 and rapa d3-5. The green fluorescent protein imaging technique has been described elsewhere (M. Yang et al., Proc Natl Acad Sci USA 97, 1206-11 (2000); M. Yang et al., Clin Cancer Res 5, 3549-59 (1999); M. Yang et al., Cancer Res 59, 781-6 (1999); M. Yang et al., Cancer Res 58, 4217-21 (1998); M. Yang, E. Baranov, A.R. Moossa, S. Penman, R. M. Hoffman, Proc Natl Acad Sci USA 97, 12278-82 (2000); and C.A. Schmitt et al., Cancer Cell 1, 289-98 (2002)). Images taken on day 21 post treatment revealed sizable tumors at several sites for both animals treated with either adriamycin or rapamycin (Figure 9B). Whole body imaging of these animals treated with either adriamycin or rapamycin alone revealed that splenic infiltration, thoracic wall lymphoma, cervical lymphoma and inguinal lymphoma persisted after 21 days following treatment. In marked contrast, the combination of chemotherapy and rapamycin showed potent activity against Akt-expressing lymphomas. The animal treated with the combination of drugs appeared to be free of tumors.

### Example 5: TUNEL and Ki67 staining of histological sections from Akt and Bcl-2 tumors

Untreated tumors produced using pMSCV-IRES-GFP (*Eµ-myc*/*wt*), pMSCV-Akt-IRES-GFP (*Eµ-myclAkt*) or pMSCV-Bcl-2-IRES-GFP (*Eµ-myc*/*Bcl-*2) were fixed in neutral buffered formaldehyde (4% in PBS) for 24 hrs, then transferred into neutral PBS. Sectioning was by standard microtome. Immunohistochemical (IHC) staining was done by addition of a) TUNEL reagent to demonstrate apoptotic cells and b) Ki67 as a marker of proliferation, using NCL-Ki67p antibody from Novocastra (Vector Labs) at 1:5000.

As shown in Figure 2C, cells of all three genotypes showed extensive staining with antibodies to the Ki67 antigen, indicating a high rate of proliferation. In contrast, TUNEL analysis showed much less DNA fragmentation or apoptosis occurring in *Eµ-myc*/*Akt* or *Eµ-myc*/*Bcl-2* cells relative to *Eµ-myc*/*wt* control cells.

### Example 6: S6-Kinase inhibition following rapamycin treatment in vitro

Cells for this immunofluorescent antibody binding study were derived from the offspring of a cross of an *Eµ-myc* mouse to a PTEN^{+/-} mouse. These mice develop *Eµ-myclPTEN*^{+/-} tumors. Cells of these tumors can be grown in culture, unlike *Eµ-myc*/Akt cells. *Eµ-myc*/*PTEN*^{*+*/*-*} tumor cells were incubated with 10 nM rapamycin (Sigma) for 1 hour, then washed in PBS. Cytospin was performed using 1×10⁵ cells at 500 rpm. Cells were then fixed in 4% paraformaldhyde for 15 minutes, washed and incubated with primary antibody to S6-kinase (Cell Signaling Technology), then washed and incubated with secondary antibody. For fluorescence detection, we used an Axioscop 50 immunofluorescence microscope (Zeiss, Thornwood, NY). DAPI (4', 6-diamidino-2-phenylindole dihydrochloride; Sigma) was added with the antibody to stain all cells (blue stain). Untreated cells fluoresced brightly, but cells treated with rapamycin showed almost no fluorescence. Figure 24 shows the marked inhibition of S6-kinase (red staining) following rapamycin treatment in vitro.

### Insert immunofluorescent figure of PTEN cells treated with RAP

### Example 7: Western blots were used to test for the effect of rapamycin on phosphorylation of elongation factor eIF4G, a target of mTOR.

Cells of one primary lymphoma were used to inject three recipient mice, and secondary lymphomas were allowed to develop. The mice were treated with 1) nothing, as control, 2) rapamycin at 4 mg/kg, or 3) rapamycin at 4 mg/kg and cyclophosphamide at 300 mg/kg i.p.).

A control experiment was performed using 3T3 fibroblasts. Fibroblasts were incubated in 1) medium without serum (cells serum-starved for 48 hours), 2) medium with serum (known to activate the PI3 kinase/Akt pathway) or 3) medium with serum, with addition of 10 nM rapamycin for 1 hour.

Protein extracts were prepared, samples were separated by electrophoresis on SDS-polyacrylamide gels, and the proteins were transferred to a membrane for probing with antibodies against phospho-eIF4G (Cell Signaling Technology).

Both the rapamycin alone and the rapamycin plus cyclophosphamide combination treatment inhibited phosphorylation of elongation factor eIF4G in the lymphoma cells. Rapamycin also inhibited phosphorylation of elongation factor eIF4G in 3T3 fibroblasts. Growth in serum increased the level of phosphorylated eIF4G compared to the level of phosphorylated eIF4G in 3T3 cells grown without serum (see Figure 8A).

### Example 8: Akt and Bcl-2 accelerate lymphomagenesis and cause drug resistance in vivo

See "*In Vivo* Chemotherapy" above for procedures. Mice reconstituted with *Eµ-myc* transgenic HSCs transduced with pMSCV ("empty vector"), Akt or Bcl-2 were followed for time to development of lymphomas. Like Bcl-2 (Schmitt, C. A., et al., "Genetic analysis of chemoresistance in primary murine lymphomas," Nat. Med. 6: 1029-1035 (2000)), Akt dramatically accelerated lymphomagenesis relative to controls (Figure 2, p < 0.0001 for both Akt and Bcl-2 relative to MSCV). Of note, a kinase-dead mutant Akt (K179A) did not cause acceleration of lymphogenesis, and Akt did not cause tumor formation in non-transgenic HSCs.

Sections of *Eµ-myclAkt* lymphomas were stained with hematoxylin and eosin (H/E), and immunohistochemical (IHC) stains for B220/CD45R and phosporylated Akt. Compared to lymphomas arising in *Eµ-myc* mice, the Akt and Bcl-2 lymphomas were more invasive and often associated with leukemia (Fig 2D, data not shown). Microphotographs of H/E stained liver sections revealed perivascular infiltration in control tumors and parenchymal invasion in Akt and Bcl-2 tumors. Immunophenotyping revealed that Akt and Bcl-2 lymphomas were derived from a B220/CD45R-positive progenitor cell which lacked other markers of B-cell differentiation, whereas control lymphomas were typically derived from a more mature B-cell type (see Fig. 2B). As shown in Figure 15, representative flow cytometric immunophenotyping of control (*myc*), Bcl-2 (*myclBcl-2*), Akt (*myc*/*Akt*) and eIF4E (*myc*/*eIF4E*) tumors, tumor cells were gated on the basis of forward scatter and side scatter and GFP positivity. Both Akt and Bcl-2 tumors did not express B-cell antigens other than CD45R (B220) but were positive for CD4, whereas the control and eIF4E tumors had a mature B-cell marker profile. At least three tumors of each genotype were analyzed. See Table 2.

**Table 2**

| Immunophenotype of tumors expressing *Eµ-myc* (Control), *Eµ-myc*/*Bcl-2, Eµ-myc*/*Akt* and *Eµ-myc*/*eIF4E* | | | | |
|---|---|---|---|---|
| **Antigen** | **Control** | **Bcl-2** | **Akt** | **eIF4E** |
| B-cell antigens | | | | |
| CD45R | Pos | Pos | Pos | Pos |
| CD19 | Pos | Neg | Neg/Weak | Pos |
| IgM | Pos | Neg | Neg | Variable |
| IgK | Pos | Neg | Neg | Pos |
| CD23(FcεR) | Neg | Neg | Neg | Neg |
| CD138 | Variable | Neg/Weak | Neg | Variable |

| T-cell antigens | | | | Neg |
|---|---|---|---|---|
| CD3 | Neg | Neg | Neg | Neg |

| **Antigen** | **Control** | **Bcl-2** | **Akt** | **eIF4E** |
|---|---|---|---|---|
| TCR α/β | Neg | Neg | Neg | Neg |
| TCR γ/δ | Neg | Neg | Neg | Neg |
| CD4 | Neg | Pos | Pos | Neg |
| CD5 | Neg | Neg | Pos | Neg |
| CD8a | Neg | Neg/Weak | Neg | Variable |

| Myeloid antigens | | | | |
|---|---|---|---|---|
| Ly-6G (Gr-1) | Neg | Pos | Neg | Neg |
| CD11b (Mac-1) | Neg/Weak | Neg/Weak | Weak | Variable |
| Ly-76 (TER119) | Neg | Neg | Neg | Neg |
| Ly-71 (F4/80) | Neg | Neg | Pos | Neg |
| CD41 | Neg | Neg | Neg | Neg |

| Lineage non-specific antigens | | | | |
|---|---|---|---|---|
| CD45 | Pos | Pos | Pos | Pos |
| *Early antigens* | | | | |
| CD34 | Neg | Neg | Neg/Weak | Neg |
| Ly-6A/E (Sca-1) | Neg | Pos | Pos | Neg |
| CD90 (Thyl) | Variable | Pos | Variable | Neg |
| CD117 (c-Kit) | Neg | Neg | Neg | Neg |

| *Additional antigens* | | | | |
|---|---|---|---|---|
| CD16/32 (FCγRII/III) | Pos | Variable | Pos | Pos |
| CD31 (PECAM) | Pos | Pos | Pos | Pos |
| CD43 | Pos | Pos | Variable | Pos |
| CD59 (Ly-6C) | Neg/Weak | Pos | Pos | Variable |
| CD69 | Neg | Neg | Neg | Pos |
| CD71 (Transferrin Receptor) | Pos | Pos | Pos | Pos |
| CD86 | Variable | Pos | Pos | Pos |
| Class II (I-a) | Pos | Pos | Pos | Pos |

Although lymphomas of all genotypes proliferated at similar rapid rates as assessed by Ki-67 staining, Akt and Bcl-2 lymphomas showed much less apoptosis relative to controls as measured by TUNEL (see Figure 2C). The impact of Akt on tumor behavior was reminiscent of *p53* tumor suppressor gene loss, which normally acts to limit Myc-induced lymphomagenesis by promoting apoptosis (Schmitt, C. A., et al., "Genetic analysis of chemoresistance in primary murine lymphomas," Nat. Med. 6: 1029-1035 (2000)). In fact, whereas all lymphomas arising *from p53+*/*- Eµ-myc* hematopoietic stem cells lost the wild-type *p53* allele, those expressing either Bcl-2 or Akt did not (see Figure 16). Tumorigenesis and acts in a manner that is temporally and pathologically similar to a strictly anti-apoptotic gene.

C57BL/6 mice harboring different transplanted lymphomas were treated with cyclophosphamide (Cytoxan, CTX) or doxorubicin (DXR) and monitored for tumor free and overall survival (see Figures 2E and F and Figure 17). Here, animals harboring *ARF*-null lymphomas were used as controls, since these lymphomas are also highly aggressive but remain chemosensitive (Schmitt, C. A., et al., "Dissecting p53 tumor suppressor functions in vivo," Cancer Cell 1: 289-298 (2002)). Following CTX therapy, mice harboring control lymphomas invariably entered a complete remission and > 50% remained tumor free after 100 days. In contrast, mice harboring Akt lymphomas responded poorly, typically displaying remissions of less than 20 days with no long-term survivors (p < 0.001 relative to control lymphomas). Although DXR produced fewer long lasting remissions in the control cohort than CTX, mice harboring Akt lymphomas still displayed substantial reductions in tumor free and overall survival (Figure 2F and Figure 17B, p < 0.0001 for Akt and Bcl-2 vs. control). Therefore, Akt-mediated survival signaling can promote drug resistance *in vivo.*

### Example 9: Inhibition of mTOR sensitizes Akt tumors to DNA damage-induced apoptosis.

We asked whether pharmacologically inhibiting the pathway downstream of Akt might have anti-tumor effects. One established Akt effector is mTOR, a serine/threonine kinase implicated in translation control, which can be potently inhibited by the immunosuppressant rapamycin (reviewed in Huang, S. and Houghton, P. J., targeting mTOR signaling for cancer therapy. Curr Opin. Pharmacol. 3: 371-377 (2003)). Although not considered a primary component of Akt survival signaling, mTOR can mediate metabolic changes important for cell survival in growth factor poor environments (Plas, D. R., et al., "Akt and Bcl-xL promote growth factor-independent survival through distinct effects on mitochondrial physiology," J. Biol. Chem. 276: 12041-12048 (2001) and Edinger, A. L. and Thompson, C. B., "Akt maintains cell size and survival by increasing mTOR-dependent nutrient uptake," Mol. Biol. Cell 13: 2276-2288 (2002)). Rapamycin has modest anti-tumor activity against PTEN-deficient tumors in animal studies and can potentiate drug-induced cell death *in vitro* (Neshat, M. S., et al., "Enhanced sensitivity of PTEN-deficient tumors to inhibition of FRAP/mTOR," Proc. Natl. Acad. Sci. USA 98: 10314-10319 (2001); Grunwald, V., et al., "Inhibitors of mTOR reverse doxorubicin resistance conferred by PTEN status in prostate cancer cells." Cancer Res. 62: 6141-6145 (2002); Podsypanina, K., et al., "An inhibitor of mTOR reduces neoplasia and normalizes p70/S6 kinase activity in Pten+/- mice," Proc. Natl. Acad. Sci. USA 98: 10320-10325 (2001); and Hosoi, H., et al., "Rapamycin causes poorly reversible inhibition of mTOR and induces p53-independent apoptosis in human rhabdomyosarcoma cells," Cancer Res. 59: 886-894 (1999)). We therefore asked whether rapamycin was effective against our defined murine lymphomas, and whether its activity was genotype-dependent or enhanced by chemotherapy.

To determine whether rapamycin inhibits mTOR in *Eµ-myc* lymphomas, its activity was indirectly assessed in extracts from untreated or treated lymphomas using antibodies that specifically recognize the phosphorylated forms of two mTOR targets, the ribosomal S6 protein (which is phosphorylated by the S6 kinase) and eIF4G.

Animals harboring lymphomas of the indicated genotypes were either not treated or treated with DXR, rapamycin or DXR and rapamycin. Lymphoma cells were harvested 7 hours post treatment. Lysates were subjected to immunoblotting for phosphorylated and total ribosomal S6 protein, phosphorylated and total eIF4G, Akt, Bcl-2, and α-tubulin as a loading control. The treated and untreated lymphomas were TUNEL-stained for apoptotic cells. As expected, untreated Akt lymphomas expressed substantially more phosphorylated S6 and eIF4G relative to Bcl-2 lymphomas (see Figure 8A, compare lanes 1 and 5). Treatment with rapamycin alone or in combination with chemotherapy substantially reduced S6 and eIF4G phosphorylation in Akt lymphomas (compare lanes 1 to 3 and 4). Hence, rapamycin inhibits mTOR activity *in vivo* in a manner that is not affected by conventional therapies.

### Example 10: Rapamycin reverses apoptotic defects and chemoresistance in Akt lymphomas

We examined the acute and long-term effects of rapamycin *in vivo.* In Akt lymphomas, rapamycin treatment produced only a slight increase in apoptosis relative to untreated counterparts by 6 hours post-therapy, as assessed by PARP cleavage in lymphoma extracts (see Figure 8A, compare lanes 1 to 3) and by TUNEL staining of lymphoma sections (see Figure 8B). Accordingly, these mice rarely achieved complete remissions (Figures 9A and B) and showed tumor free and overall survival patterns that were no better than those produced by conventional therapy (Figure 9C and Figure 11). Furthermore, rapamycin displayed even less activity against lymphomas of other genotypes (Figures 8 and 9). Therefore, despite its ability to inhibit mTOR *in vivo,* rapamycin had only modest activity as a single agent.

In marked contrast, the combination of chemotherapy and rapamycin showed potent activity against Akt-expressing lymphomas. For example, DXR and rapamycin treatment produced PARP cleavage and massive apoptosis 6 hours post-therapy (Figure 8A, compare lanes 1 and 4; Figure 8B), with all of the animals achieving complete remissions (Figure 9A and B, see Figure 10 for a comparison of matched groups). Indeed. DXR and rapamycin produced more complete remissions and better tumor free survival in the otherwise drug resistant Akt lymphomas than DXR produced in the chemosensitive controls (Figures 9A and C, p < 0.001 for RAP+DXR vs. DXR or RAP alone), resulting in a substantial survival benefit (Figure 11A, p < 0.001 for RAP+DXR vs. DXR or RAP alone). Similar effects were observed with the combination of CTX and rapamycin, where approximately half of the mice achieved remissions lasting more than 60 days compared to none for either agent alone (Figure 11B and 13A, p < 0.001). Consequently, rapamycin reverses the apoptotic defects and drug resistance occurring in Akt lymphomas.

Mice bearing matched tumors were generated by injecting 1x 10⁶ tumor cells from the same primary tumor into 3 recipient animals. Eight 'matched groups' of 3 mice received DXR, RAP, or DXR + RAP. Mice were monitored twice weekly by palpation and blood smears. Figure 10 indicates the individual tumor free survival times and the arithmetic mean; a paired t-test analysis comparing either combination to the single agents confirmed statistical significance (for DXR versus RAP+DXR: p = 0.0002; RAP versus RAP+DXR: p < 0.0001; while DXR versus RAP: p = 0.7).

The potent effects of rapamycin in combination therapy were not observed in lymphomas of other genotypes. Bcl-2 lymphomas, which showed a similar drug resistance profile to either rapamycin or chemotherapy alone, remained non-responsive to the combination as assessed in both acute and long-term assays (Figure 9A and D; Figure 11C and D; Figure 12B). Moreover, control lymphomas, which were initially chemosensitive, showed no improvement in short or long term responses following combination therapy and, in fact, rapamycin appeared to *antagonize* drug-induced apoptosis in these cells (Figure 8B). Therefore, while each lymphoma studied contained an anti-apoptotic lesion, the chemosensitizing effects of rapamycin were specific for tumors with constitutive Akt signaling.

### Example 11: eIF4E is oncogenic in vivo and causes resistance to conventional and targeted therapy

The results described above suggest that mTOR is essential for Akt-mediated survival signaling. mTOR can regulate translation in response to nutrients and growth factors by phosphorylating key components of the protein synthesis machinery, including the 40S ribosomal protein S6 kinase, p70^{S6K}, and the 4E-BP proteins (Schmelzle, T. and Hall, M. N., "TOR, a central controller of cell growth," Cell 103: 253-262 (2000)). Phosphorylation of 4E-BP, in turn, releases the translation initiation factor eIF4E to stimulate cap-dependent translation. Although its role in oncogenesis is poorly understood, eIF4E can have transforming and anti-apoptotic activities *in vitro* (Lazaris-Karatzas, A., et al., "Malignant transformation by a eukaryotic initiation factor subunit that binds to mRNA 5' cap," Nature 345: 544-547 (1990) and Polunovsky, V. A., et al. "Translational control of the antiapoptotic function of Ras," J. Biol. Chem. 275: 24776-24780 (2000)) and is overexpressed in many tumor types (Hershey, J.W.B. and Miyamoto, S. In: Translational Control of Gene Expression, N. Sonenberg et al., eds., Cold Spring Harbor, NY, (2000) pp. 637-654). Furthermore, Akt-expressing tumors selectively increase the translation of some mRNAs in a rapamycin-reversible manner (Grolleau, A., et al., "Global and specific translational control by rapamycin in T cells uncovered by microarrays and proteomics," J. Biol. Chem. 277: 22175-22184 (2002) and Rajasekhar, V. K., et al., "Oncogenic Ras and Akt signaling contribute to glioblastoma formation by differential recruitment of existing mRNAs to polysomes," Mol. Cell. 12: 889-901 (2003)). Therefore, to further examine the effects of translational control on tumor phenotypes, we directly compared eIF4E to Akt in the *Eµ-myc* model (see Figure 1).

The time to tumor development was observed, following hematopoietic reconstitution with *Eµ-myc* transgenic HSCs expressing eIF4E (n = 15) versus control *Eµ-myc* transgenic HSCs infected with "empty vector" pMSCV and *Eµ-myc* transgenic HSCs transduced with Akt. (See Figure 13A, same control and Akt data from Fig 2). eIF4E accelerated lymphomagenesis in a manner that was similar to Akt (Figure 13A, median onset of 50 and 42.5 days for Akt and eIF4E, respectively, p<0.0001 for eIF4E vs. control). Of note, eIF4E did not induce tumors in a non-transgenic background within the observation period (> 125 days, data not shown).

Organs (lymph node, liver, kidney/renal pelvis) from mice bearing *Eµ-myc*/eIF4E lymphomas were sectioned and stained with hematoxylin and eosin (H/E) (Figure 13B, top). Untreated tumors were examined by IHC stains for phosphorylated Akt and Ki-67, and by TUNEL, using sections of tumors harvested 6-8 hours following the therapy (Figure 13B, bottom). eIF4E could also compensate for *p53* loss during lymphomagenesis, as lymphomas arising from *p53+*/*- Eµ-myc* hematopoietic stem cells retained the wild-type *p53* allele (Figure 16). Despite these similarities, eIF4E lymphomas were derived from a more mature B-cell type and often produced an aggressive infiltration of the renal pelvis distinct from Akt-associated pathologies (Figures 15 and 13B). Nevertheless, eIF4E is clearly a potent oncogene *in vivo,* producing phenotypes consistent with an anti-apoptotic gene.

Moreover, like Akt lymphomas, eIF4E lymphomas displayed a disseminated pathology and a high proliferation/apoptosis ratio, despite only a ∼2.5 fold increase in eIF4E protein (see Figure 13C, compare lanes 1 to 3 and Figure 14). Lysates of eIF4E and Akt tumors, harvested without prior therapy (U) or 6 hours after rapamycin (R) administration *in vivo* were subjected to immunoblotting for eIF4E binding protein 1 (4E-BP1), the phosphorylated and total ribosomal S6 protein (p-S6 and S6), total eIF4E, phosphorylated and total Akt (p-Akt and Akt) proteins, and actin as a loading control (see Figure 13C). Note that untreated Akt tumors (lane 3) show an increase in the phosphorylated, slower migrating forms of 4E-BP1.

Allele-specific PCR was carried out to detect the wild-type (*p53* WT) and mutant allele (*p53* Neo) in tumors derived from *Eµ-myc*/*p53*^{*+*/*-*} HSCs. All control tumors showed loss of heterozygosity (LOH) (8/8), while none of the Akt (0/5), Bcl-2 (0/5) or eIF4E (0/3) tumors underwent LOH (see Figure 16).

In flow cytometric immunophenotyping of control (m*yc*), Bcl-2 (*myc*/*Bcl-*2), Akt (*myc*/*Akt*) and eIF4E (*myc*/*eIF4E*) tumors, tumor cells were gated on the basis of forward scatter and side scatter and GFP positivity. At least three tumors of each genotype were analyzed. Both Akt and Bcl-2 tumors did not express B-cell antigens other than CD45R (B220) but were positive for CD4, whereas the control and eIF4E tumors had a mature B-cell marker profile (see Figure 15).

Lysates derived from Bcl-2 (n = 3), Akt (n = 5) and eIF4E (n = 5) tumors were probed with antibodies against eIF4E and β-actin as loading control and I¹²⁵ labeled Protein A to quantitate eIF4E expression. Radioactivity was quantitated on western blots by phosphorimaging. Figure 14 is a graphic representation of eIF4E expression relative to Akt and Bcl-2 tumors. The presented data are the combined averages of 4 independent probings of the tumor set with error bars denoting the standard deviation. Bcl-2 (n = 4), Akt (n = 5), eIF4E (n = 5).

Lymphomas expressing eIF4E were highly resistant to DXR therapy relative to controls, and mice harboring these lymphomas displayed tumor free and overall survival patterns that were indistinguishable from those harboring Akt lymphomas (Figure 13E, p< 0.0001 vs. Control; p=0.47 vs. Akt). However, whereas Akt lymphomas displayed high mTOR activity as assessed by an increase in the rapamycin-sensitive phosphorylation of S6 and 4E-BP1, eIF4E lymphomas did not (Figure 13C). However, in contrast to Akt lymphomas, eIF4E lymphomas displayed no increase in the rapamycin-sensitive S6 phosphorylation and 4E-BP1 (Figure 13C) and were non-responsive to rapamycin/DXR therapy *in vivo* (Figure 13E, Figure 11E, p<0.0001). Furthermore, introduction of eIF4E into an initially sensitive Akt lymphoma conferred resistance to rapamycin and chemotherapy; hence cells co-expressing eIF4E were enriched in mixed populations following therapy relative to over those expressing Akt alone (Figure 13F and G). Together, these results indicate that eIF4E can recapitulate Akt action in oncogenesis and drug resistance. They also show that eIF4E can confer resistance to a rapamycin based therapy *in vivo,* presumably because it acts downstream of mTOR.

### Example 12: Rapamycin reverses apoptotic defects and chemoresistance in PTEN-deficient lymphomas

Generation and development of PTEN mutants (see Di Cristofano A., et al., "Pten is essential for embryonic development and tumor suppression", Nature Genetics, 19(4):348-355 (1998); Suzuki A., et al., "High cancer susceptibility and embryonic lethality associated with mutation of the PTEN tumor suppressor gene in mice", Current Biology, 8(21):1169-1178, (1998)).

*Eµ-myc*/PTEN+/- mice were derived from a cross of a transgenic *Eµ-myc* mouse to a transgenic *PTEN+*/*-* mouse and detection of loss of heterozygosity (LOH) in the *PTEN* locus was by allele specific PCR (Di Cristofano A., et al., Nature Genetics, 19(4):348-355, 1998).

The time from birth to tumor development was observed in *Eµ-myc*/PTEN+/- mice (n=12) and control *Eµ-myc*/PTEN+/+ mice (n=24). The animals were monitored 2-3 times weekly for the occurrence of well-palpable peripheral lymph node enlargements. Tumors arising in *Eµ-myc*/*PTEN+*/*-* mice were harvested and either fixed for histological evaluation, rendered single cell suspensions and frozen in 10% DMSO or transplanted directly into normal C57BL/6 (non-transgenic) mice for treatment studies (Schmitt, C. A., et al., "Genetic analysis of chemoresistance in primary murine lymphomas," Nat. Med. 6: 1029-1035 (2000)).

Initial time to tumor onset in *Eµ-myc*/PTEN+/- and *Eµ-myc*/PTEN+/- mice is shown in Figure 18. Time to onset data were calculated from birth to lymphoma onset (cumulative survival versus time). Statistical evaluation of tumor onset data was by log-rank (Mantel-Cox) test for comparison of the Kaplan-Meier event-time format. Results show that PTEN heterozygous mice are highly susceptible to tumors, as heterozygosity caused a significant acceleration and increased penetrance in lymphoma development in vivo (p<0.005).

Primary tumors arising in *Eµ-myc*/PTEN+/- and *Eµ-myc*/PTEN+/- mice were harvested and injected into C57BL/6 (non-transgenic) mice. Upon tumor formation of palpable secondary tumors, mice were treated with a single i.p. administration of drug. The treatment schedule included an injection with either DXR (10 mg/ml in 0.9% saline), CTX (300 mg/kg in 0.9% saline), RAP (4 mg/kg in 0.9% H₂O with 5% PEG/TWEEN), or a combination thereof (d1: DXR or CTX, d2: DXR or CTX and RAP, d3-5: DXR pr CTX). Mice were then palpated twice weekly for disappearance or recurrence of tumors. Data are presented as Kaplan-Meier plots in Figure 19, and show the tumor free survival/time to relapse. Time to relapse for mice that were never tumor free was scored as '0' days. Tumor free survival implies the complete absence of detectable disease, i.e. no frank leukemia and no palpable tumors, a relapse denotes the recurrence of either, e.g. following RAP+DXR treatment (green line), all mice achieve a complete remission, by day 15 the first animal has relapsed and by day 23 all mice have relapsed. The bottom panel has an equivalent analysis for treatment with CTX, RAP, and RAP+CTX combination. Together these data show that *Eµ-myc*/PTEN+/- tumors are resistant to DXR and RAP reverses this drug resistance.

### Example 13: eIF4E blocks rapamycin's effect on chemoresistance in PTEN-deficient lymphomas

Parent vector pMSCV-IRES-GFP, or derivative vector pMSCV-eIF4E-IRES-GFP were used to transduce hematopoietic stem cells obtained as described in prior examples. Recipient *Eµ-myc*/PTEN+/- and *Eµ-myc*/PTEN-/- mice were administered the transduced hematopoietic stem cells as described previously.

Stem cells were infected using retroviral vectors (either pMSCV-IRES-GFP as control, or pMSCV-eIF4E-IRES-GFP). When primary tumors arose in these animals, the mice were sacrificed and the tumors were harvested and injected into recipient C57BL/6 (non-transgenic) mice. Following injection of 10⁶ cells into the tail vein, upon development of palpable (secondary) tumors (approximately 3 weeks following the i.v. injection), drugs were administered. Mice were treated once by i.p. injection with either DXR (10 mg/ml in 0.9% saline), CTX (300 mg/kg in 0.9% saline), RAP (4 mg/kg in 0.9% H₂O with 5% PEG/TWEEN), or a combination wherein the combination schedule was d1: DXR or CTX, d2: DXR or CTX and RAP, d3-5: DXR pr CTX). The tumors were harvested 48 hours after treatment and a single cell suspension was analyzed for GFP expression by flow cytometry as described previously. GFP expression denotes tumor cells which are productively infected with MSCV-eIF4E-IRES-GFP.

Figure 20 shows the GFP content measured by flow cytometry under each treatment condition (untreated, DXR, RAP, RAP + DXR). The observed increase in GFP-positive cells in all treated tumors as compared to the untreated control indicates an advantage/resistance of eIF4E-infected cells under different therapies.

### Example 14: Tumor free survival of different forms of mutated eIF4E cells influence eIF4E's oncogenicity in vitro

Day 13.5-18.5 pregnant mice from an *Eµ-myc*-transgenic to wildtype (C57BL/6) cross were sacrificed to obtain fetal livers, which were minced and grown at approximately 3×10⁶ cells/ml in conditions supporting hematopoietic stem cell (HSC) growth (37% DMEM, 37% Iscove's modified Dulbecco's Medium [Gibco], supplemented with 20% fetal calf serum, 2% L-glutamine [200 mM], 100 U/ml penicillin/streptomycin, 5×10⁻⁵ M 2-mercaptoethanol, 4% 0.45 µm filtered WEHI-3B supernatant, 0.2 ng/ml recombinant murine interleukin-3, 2 ng/ml recombinant murine interleukin-6, and 20 ng/ml recombinant murine stem cell factor [all cytokines from Research Diagnostics] at 37°C in a humidified 5% CO₂ atmosphere).

*Eµ-myc* HSCs were harvested and infected in vitro, as described earlier, with MSCV-IRES-GFP constructs expressing different mutants of eIF4E. The mutants were: (a) E103A; an amino acid involved in ionic interaction with the guanosine ring (bearing a partial positive charge near the N-7 methyl portion of the guanosine ring), (b) S209A; which alters a MNK phosphorylation site (see Pyronnet S. et al., "Human eukaryotic translation initiation factor 4G (eIF4G) recruits Mnk1 to phosphorylate eIF4E", EMBO J.18, 270-279 (1999)). Such a mutation does not affect the formation of initiation complex, mitogen-stimulated increase in cap-dependent translation, and cell proliferation. It is thought that through the kinase activity of MNKs, eIF4E activity is limited under physiological conditions. MNK1 may define a convergence point between the growth factor-activated and one of the stress-activated protein kinase cascades and is a candidate to phosphorylate eIF4E in cells, (c) W56A; which should not bind the cap-structure, (d) W73A; an important residue in the interaction of eIF4E and eIF4E binding protein (4E-BP). Changing this amino acid is known to prevent productive interaction with 4E-BP and eIF4G.

Figure 21 shows that E103A induces tumors with high penetrance and only slightly slower than wild type eIF4E, while the mutants W56A and W73A appear to be inactive. Moreover, S209A induces tumors at lower penetrance and much delayed. This would indicate that the phosporylation site S209 is important for eIF4E's oncogenicity. As phosphorylation at this site is mediated via p38 and MEKK activation of MNK-1, this finding also indicates that eIF4E tumors (and similarly Akt expressing tumors) should be sensitive to pharmacological inhibitors of these kinases.

### Example 15: Presence of novel PTEN short hairpins induces chemoresistance and downregulation of PTEN expression in vitro

Retroviral-mediated gene transfer was performed using Phoenix packaging cells (a gift from G. Nolan, Stanford University) as previously described (Serrano M., et al., "Oncogenic ras provokes premature cell senescence associated with accumulation of p53 and p161NK4A", Cell 88:593-602, (1997)). The two murine PML hairpins (SEQ ID NO:1 and SEQ ID NO:2) were cloned into MSCVpuro (Clontech) as previously described (Hemann M.T., et al., "An epiallelic series of p53 hypomorphs created by stable RNAi produces distinct tumor phenotypes in vivo", Nature Genetics 33:396-400, (2003)). Infected cell populations were selected in puromycin (2 µg/ml, 2 days).

Initial time to tumor onset in *Eµ-myc* mice infected with a short-hairpin against PTEN (MSCV-shPTEN-IRES-GFP) is shown in Figure 22. Time to onset data were calculated following reconstitution of lethally irradiated mice with HSCs from the fetal livers of *Eµ-myc* mice infected with a short hairpin against two mPTEN sequences (PTEN sh1 and PTEN sh2) (n=5). Data is presented as cumulative survival versus time. Control group are equivalent HSCs infected with a vector encoding only GFP (MSCV-IRES-GFP) (n=25). Statistical evaluation of tumor onset data was by log-rank (Mantel-Cox) test for comparison of the Kaplan-Meier event-time format. Results show that shPTEN induces lymphomas in the *Eµ-myc* mouse model.

Wild type Mouse Embryo Fibroblasts (wt MEFs) containing a control vector (MSCV-IRES-GFP) or a short hairpin against PTEN sh1 and PTEN sh2 were collected and analyzed for the expression of endogenous total and phosphorylated PTEN by western blotting. Immunoblots were performed from whole-cell lysates obtained by boiling cell pellets solubilized in Laemmli sample buffer (de Stanchina E., et al., "E1A signaling to p53 involves the p19ARF tumor suppressor.", Genes Dev., 12:2434-2442, (1998)). Samples of 30 mg of protein (Bio-Rad protein assay) were separated on SDS-PAGE gels and transferred to Immobilon-P membranes (Millipore). The antibodies used were: anti-PTEN 488 ( 1: 1000 dilution, a gift from M. Myers) and anti-PTEN phosphoSer380 (1:1000 dilution, Cell Signaling). Proteins were visualized using ECL (Amersham) or SuperSignal West Femtomaximum (Pierce). As shown in Figure 23, PTEN expression is downregulated in the presence of PTEN short hairpins.

### Example 16: Tumor free survival of eIF3E-expressing mice in vivo

Retroviral infections using MSCV vectors were carried out as described previously. Infected HSC populations were propagated in recipient mice as described previously. Parent vector pMSCV-IRES-GFP was constructed as described previously. Derivative vector pMSCV-eIF3E-IRES-GFP was constructed with a gene encoding the p48 fragment of eIF3E. The eIF3E gene (GenBank accession #: 7: XM_129062) was cloned from pMV7 by excising EcoRI and HindIII sites of pMV7 (Mayeur, G.L. and Hershey, W.B., ''Malignant transformation by the eukaryotic translation initiation factor 3 subunit p48 (eIF3E)", FEBS Letters, 514: 49-54 (2002)). MSCV-IRES-GFP was then cut with these same enzymes (and as described previously) and eIF3E was ligated into MSCV.

The tumor free survival of *Eµ-myc* mice infected with p48/eIF3E (MSCV-p48/eIF3E-IRES-GFP) is shown in Figure 25. Time to onset data were calculated following reconstitution oflethally irradiated mice with HSCs from the fetal livers of *Eµ-myc* mice infected with a short hairpin against p48/eIF3E (n=16). Data is presented as cumulative survival versus time. Control group are equivalent HSCs infected with a vector encoding only GFP (MSCV-IRES-GFP) (n=25). Statistical evaluation of tumor onset data was by log-rank (Mantel-Cox) test for comparison of the Kaplan-Meier event-time format. Results show that p48/eIF3E induces lymphomas in the *Eµ-myc* mouse model.
SEQ ID NO:1
   Mouse PTEN hairpin 1
SEQ ID NO:2
   Mouse PTEN hairpin 2

## Claims

1. A method for identifying a treatment that increases sensitivity of a hematopoietic tumor cell to a chemotherapeutic agent, the method involving:
(a) providing a test mouse that has been engineered to develop a hematopoietic tumor, wherein the mouse comprises a population of recombinant hematopoietic host cells in which the P13K pathway has been activated by expression of a vector comprising an akt gene, or by expression of a short-hairpin RNA (shRNA) against PTEN (shPTEN), the mouse having been administered a chemotherapeutic agent and a test treatment;
(b) monitoring the mouse for the development of hematopoietic tumors; and
(c) comparing the extent to which such tumors are present in the mouse after the test treatment compared to the extent to which such cells are present in a control mouse maintained under the same conditions as the test mouse except that no test treatment is administered to the control mouse; wherein:
(i) if tumor onset occurs less frequently in the test mouse than in the control mouse, or if remission from the primary hematopoietic tumors occurs more frequently in the test mouse than in the control mouse, the treatment is one which increases chemosensitivity, and
(ii) if tumor onset occurs more frequently in the test mouse than in the control mouse, or if remission from the primary hematopoietic tumors occurs less frequently in the test mouse than in the control mouse, the treatment is one which decreases chemosensitivity.

2. The method of claim 1, wherein the vector is a pMSCV-Akt vector.

3. The method of claim 1, wherein the vector is a pMSCV-Akt-IRES-GFP vector.

4. The method of claim 1, wherein Akt expression is constitutive.

5. The method of claim 1, wherein the chemotherapeutic agent is selected from the group consisting of cyclophosphamide and doxorubicin.

6. The method of claim 1, wherein the recipient mouse is a Eµ-myc transgenic mouse.

## Patentansprüche

1. Verfahren zum Identifizieren einer Behandlung, die die Empfindlichkeit einer hämatopoetischen Tumorzelle gegenüber einem chemotherapeutischen Mittel erhöht, wobei das Verfahren Folgendes umfasst:
(a) Bereitstellen einer Testmaus, die dafür entwickelt wurde, einen hämatopoetischen Tumor zu entwickeln, wobei die Maus eine Population von rekombinanten hämatopoetischen Wirtszellen umfasst, in denen der P13K-Weg durch die Expression eines Vektors, der ein akt-Gen umfasst, oder durch die Expression einer kurzen Haarnadel-RNA (shRNA) gegen PTEN (shPTEN) aktiviert wurde, wobei der Maus ein chemotherapeutisches Mittel und eine Testbehandlung verabreicht wurde;
(b) Überwachung der Maus hinsichtlich der Entwicklung von hämatopoetischen Tumoren und
(c) Vergleichen des Ausmaßes, in dem diese Tumoren nach der Testbehandlung in der Maus vorhanden sind, mit dem Ausmaß, in dem diese Zellen in einer Kontrollmaus vorhanden sind, die unter den gleichen Bedingungen wie die Testmaus gehalten wurde, mit dem Unterschied, dass der Kontrollmaus keine Testbehandlung verabreicht wird, wobei:
(i) die Behandlung die Chemosensitivität erhöht, wenn der Tumor weniger häufig in der Testmaus als in der Kontrollmaus auftritt oder wenn die Remission von den primären hämatopoietischen Tumoren häufiger in der Testmaus als in der Kontrollmaus auftritt, und
(ii) die Behandlung die Chemosensitivität verringert, wenn der Tumor häufiger in der Testmaus als in der Kontrollmaus auftritt oder wenn die Remission von den primären hämatopoietischen Tumoren weniger häufig in der Testmaus als in der Kontrollmaus auftritt.

2. Verfahren nach Anspruch 1, wobei der Vektor ein pMSCV-Akt-Vektor ist.

3. Verfahren nach Anspruch 1, wobei der Vektor ein pMSCV-Akt-IRES-GFP-Vektor ist.

4. Verfahren nach Anspruch 1, wobei die Akt-Expression konstitutiv ist.

5. Verfahren nach Anspruch 1, wobei das chemotherapeutische Mittel aus der Gruppe ausgewählt ist, die aus Cyclophosphamid und Doxorubicin besteht.

6. Verfahren nach Anspruch 1, wobei die Empfängermaus eine Eµ-myc transgene Maus ist.

## Revendications

1. Procédé d'identification d'un traitement qui augmente la sensibilité d'une cellule de tumeur hématopoïétique à un agent de chimiothérapie, le procédé consistant à :
(a) fournir une souris expérimentale qui a été manipulée pour développer une tumeur hématopoïétique, la souris comprenant une population de cellules hôtes hématopoïétiques recombinées dans lesquelles la voie P13K a été activée par l'expression d'un vecteur comprenant un gène Akt, ou par l'expression d'un petit ARN en épingle à cheveux (shARN) contre PTEN (shPTEN), un agent de chimiothérapie et un traitement expérimental ayant été administrés à la souris ;
(b) surveiller la souris pour évaluer le développement de tumeurs hématopoïétiques ; et
(c) comparer la mesure dans laquelle ces tumeurs sont présentes chez la souris après le traitement expérimental par comparaison avec la mesure dans laquelle ces cellules sont présentes chez une souris témoin maintenue dans les mêmes conditions que la souris expérimentale à cette exception près qu'aucun traitement expérimental n'est administré à la souris témoin ; dans cette comparaison :
(i) si les tumeurs apparaissent moins fréquemment chez la souris expérimentale que chez la souris témoin, ou si une rémission des tumeurs hématopoïétiques primaires a lieu plus fréquemment chez la souris expérimentale que chez la souris témoin, le traitement est un traitement qui augmente la chimiosensibilité ; et
(ii) si les tumeurs apparaissent plus fréquemment chez la souris expérimentale que chez la souris témoin, ou si une rémission des tumeurs hématopoïétiques primaires a lieu moins fréquemment chez la souris expérimentale que chez la souris témoin, le traitement est un traitement qui diminue la chimiosensibilité.

2. Procédé selon la revendication 1, dans lequel le vecteur est un vecteur pMSCV-Akt.

3. Procédé selon la revendication 1, dans lequel le vecteur est un vecteur pMSCV-Akt-IRES-GFP.

4. Procédé selon la revendication 1, dans lequel l'expression du gène Akt est constitutive.

5. Procédé selon la revendication 1, dans lequel l'agent de chimiothérapie est sélectionné dans le groupe constitué du cyclophosphamide et de la doxorubicine.

6. Procédé selon la revendication 1, dans lequel la souris receveuse est une souris transgénique Eµ-myc.
